# EUROPEAN PATENT APPLICATION

(11) **EP 1 211 317 A1**
(43) Date of publication of application: **05.06.2002**
(21) Application number: 00955046.8
(22) Date of filing: 25.08.2000
(51) Int. Cl.: C12N 15/54, C12N 9/12, C12N 5/14, A01H 5/00

(54) **GENE ENCODING PROTEIN PARTICIPATING IN SIGNAL TRANSDUCTION OF CYTOKININ**

(30) Priority: 26.08.1999 JP 24043399
(71) Applicant: SUNTORY LIMITED, Osaka-shi, Osaka 530-8203 (JP); NIPPON PAPER INDUSTRIES CO., LTD., Kita-ku, Tokyo 114-0002 (JP)
(72) Inventor: KAKIMOTO, Tatsuo, Toyonaka-shi, Osaka 560-0005 (JP)
(74) Representative: Stoner, Gerard Patrick
(86) International application number: JP0005772
(87) International publication number: WO0116332

(57) **Abstract**

There is provided a gene coding for a protein involved in cytokinin signal transduction having the amino acid sequence listed as SEQ. ID. No.2, which is derived, for example, from *Arabidopsis thaliana*. The gene may be utilized for production of the protein, or it may be introduced into a plant for plant regeneration, differentiation, growth or the like.

## Description

### Technical Field

The present invention relates to a gene coding for a protein involved in cytokinin signal transduction, and to a method for its use.

### Background Art

Cytokinin is the general term for a class of plant hormones that play an important role in plant morphogenesis and physiological phenomena, and a class of derivatives having a basic skeleton with, usually, a 5C substituent bonded to the 6-position amino group of 6-aminopurine, and with various modifications on side chains. Cytokinins interact with auxins, another class of plant hormones, to control plant cell division and differentiation and new organogenesis in tissue culture systems. As for organogenesis, auxins promote formation of roots from calli, leaves and stems, while cytokinins promote bud formation. When cytokinins are given to normal plant bodies, they exhibit such effects as promotion of lateral bud growth, inhibition of root growth, and retardation of flowers, leaves, fruits and the like. When given to seeds, they exhibit an effect of promoting germination, depending on the plant species.

The various physiological effects mentioned above are believed to result from reception of cytokinins by cells, conversion to intracellular chemical signals, transduction of those signals, and control of expression of various target genes located downstream therefrom.

While regulation of the cytokinin synthesis pathway is clearly connected with control of the various physiological functions exhibited by cytokinins, little is known about the molecular mechanisms controlling synthesis of cytokinins in plants.

Phosphate group transfer by a two-component system has recently been clearly implicated in at least a part of the cytokinin signal transduction pathway (Kakimoto, Science, 274, p.982-985, 1996, Kakimoto, Saibo Kagaku [Cell Engineering], Special issue: Shokubutu Saibo Kogaku [Plant Cell Engineering] Series 10, p.75-84, 1998). The two-component system is a type of signal transduction pathway widely found in prokaryotic organisms, and it basically involves two proteins known as a sensor histidine kinase and a response regulator.

Most histidine kinases span the cell membrane at their N-terminal region, with the N-terminal region receiving the external signal, but the majority of cytoplasm-localized histidine kinases perform roles mediated by signals from receptors. When the input domain of the sensor histidine kinase detects a signal, a specific histidine residue of the histidine kinase domain is phosphorylated. The phosphate group is then transferred to a specific aspartic acid residue of the response regulator which is the other protein of the two-component system, and the presence or absence of this phosphorylation leads to the downstream signal transduction pathway and the target gene expression regulation.

In a two-component system, the phosphate group is often first transferred from the sensor histidine kinase to a protein known as a phosphate group transfer mediator, and finally to the response regulator. For example, in the osmoregulatory pathway of yeast (*Saccharomyces cerevisiae*), the phosphate group is transferred from a sensor histidine kinase, osmolarity receptor SLN1, to a phosphate group transfer mediator, YPD1, and is then transferred to a response regulator, SSK1. Prokaryotes are known to possess numerous signal transduction mechanisms via two-component systems, but recently two-component system factors have also been found to perform important functions in fungi and plants as well. These include, for example, the yeast osmoregulatory pathway and the plant ethylene signal transduction pathway.

The present inventor considered that it might be possible to elicit a cytokinin response even in the absence of cytokinins, by increasing the level of transcription of genes involved in cytokinin synthesis or in the signal transduction pathway, and thus carried out activation tagging. This is a method whereby an enhancer sequence is randomly introduced into a plant genome to activate transcription of a plant gene near the inserted enhancer in order to obtain a dominant mutant. The present inventors transformed approximately 50,000 *Arabidopsis thaliana* calli using the vector pPCVICEn4HPT (Hayashi et al., Science, 258, p.1350-1353, 1992) developed for activation tagging.

pPCVICEn4HPT contains a strong enhancer sequence derived from the enhancer of the cauliflower mosaic virus 35S RNA gene on T-DNA, and after insertion into the plant genome via *Agrobacterium,* transcription of the gene adjacent to the enhancer is activated. For example, if the T-DNA is inserted near a gene involved in a cytokinin synthesis pathway, receptor or signal transduction pathway, the transformant would be expected to exhibit a typical cytokinin response even in the absence of the cytokinin. The present inventor cultured over 50,000 calli in medium containing no plant hormones, and separated out 4 lines of transformed calli which exhibited the typical cytokinin responses of cell growth and adventitious shoot formation.

These were designated as cytokinin independent (cki1) mutants. They exhibited cell growth, adventitious shoot formation and virescence, but formed no roots and were sterile. The 4 lines had the T-DNA inserted upstream from the same gene, and as a result of analysis of the causative gene CKI1, the protein coded for by the CKI1 gene was found to have the characteristic sequence of a sensor histidine kinase of a two-component system, with two hydrophobic domains believed to be membrane spanning domains at the N-terminal end.

CKI1 has been demonstrated to elicit a typical cytokinin response when overexpressed in plants (Kakimoto, Science, 274, p.982-985, 1996). Expression of CKI1 gene in yeast have implicated in transfer of a phosphate group to the osmoregulatory two-component system factor YPD1 in yeast. This suggests that CKI1 is a cytokinin receptor with a function of converting cytokinin stimulation into the chemical signal of phosphate transfer, and transmitting it to a downstream factor.

In addition, Sakakibara et al. (Plant J., 14, p.337-344, 1998) have reported that two-component system response regulators are encoded by genes such as ZmCip1 whose expression is elicited by treatment of maize leaves with cytokinins, or IBC6 whose expression is elicited by treatment of *Arabidopsis thaliana* with cytokinins, and therefore two-component systems are also thought to be involved in the cytokinin signal transduction pathway. Such findings indicate the possibility that these response regulators act downstream from histidine kinase cytokinin receptors.

However, the constituent of the cytokinin signal transduction pathway by CKI1 or ZmCip1 proteins remains unclear. For example, it is unknown whether phosphate group transfer mediator is involved in phosphate group transfer from histidine kinase CKI1, as the candidate cytokinin receptor, to the downstream response regulator. Moreover, it is unknown whether or not cytokinin signals are transduced by a plurality of cytokinin receptors in independent signal transduction pathways acting in parallel, and whether or not such a plurality of signal transduction pathways interact with and affect each other, and this remains as an issue to be resolved in the future.

### Disclosure of the Invention

It is therefore an object of the present invention to provide a novel factor of a two-component system involved in the cytokinin signal transduction pathway, a gene coding therefor, a protein encoded by the gene, and uses therefor.

The present inventor carried out activation tagging using *Arabidopsis thaliana* and obtained a novel gene coding for a two-component system factor involved in the cytokinin signal transduction pathway.

The present invention provides, therefore, a gene coding for a protein involved in the cytokinin signal transduction pathway. Specifically, the gene codes for a protein with histidine protein kinase activity in a two-component system. The protein encoded by the gene is predicted to be soluble in the cytoplasm, and no gene coding for a histidine protein kinase with such properties has yet been reported for plants.

More specifically, the invention provides a gene coding for a protein having the amino acid sequence listed as SEQ. ID. No.2 and involved in cytokinin signal transduction. The invention further provides a gene coding for a protein having the amino acid sequence listed as SEQ. ID. No.2 modified with one or more amino acid additions, deletions and/or substitutions with other amino acids, and having activity involved in cytokinin signal transduction. The invention still further provides a gene which hybridizes with the nucleic acid listed as SEQ. ID. No.1, and particularly DNA or a portion thereof, and which codes for a protein involved in cytokinin signal transduction and particularly a protein having histidine protein kinase activity.

The invention still further provides a vector containing the aforementioned gene.

The invention still further provides a host transformed by the aforementioned vector. The host may be plant cells or a plant body.

The invention still further provides a method of producing a protein involved in cytokinin signal transduction by culturing or cultivating the aforementioned host.

The invention still further provides a method of regulating growth of a plant or plant cells by introducing the aforementioned gene into the plant or plant cells and by expressing the gene. In other words, by expressing this gene it is possible to regulate various physiological functions associated with cytokinins, such as adventitious shoot formation promotion, breakage of lateral bud dormancy, retardation of flower and leaf aging and fruit maturation, improvement of flower lifespan and maintenance of photosynthetic function, promotion of fruit enlargement and prevention of fruit abscission, flower formation control, etc.

### Brief Description of the Drawings

Fig. 1 is an illustration showing the structure of plasmid pBI35T.
Fig. 2 is an illustration showing the structure of plasmid pBI35T.CKI2.163.
Fig. 3 is an illustration showing the structure of plasmid pBI35T.CKI2.162.
Fig. 4 is an illustration showing the structure of plasmid pEL2 omega35T.
Fig. 5 is an illustration showing the structure of plasmid pEL2 omega35T.CKI2.163.
Fig. 6 is an illustration showing the structure of plasmid pEL2 omega35T.CKI2.162.

### Best Mode for Carrying Out the Invention

The present inventor considered that it might be possible to obtain a mutation introduced plant with a new protein gene involved in the cytokinin signal transduction pathway by activation tagging in the same manner as when isolating the CKI1 gene. *Arabidopsis thaliana* transformant calli having vector pPCVICEn4HPT for activation tagging introduced via *Agrobacterium* were screened on cytokinin-free medium, and transformants that formed adventitious shoots in the absence of cytokinins were separated. Of these, a transformed callus named cki2 exhibited abundant cell growth and formed adventitious shoots in the absence of cytokinins, unlike the wild-type callus.

As a result of isolating and analyzing the CKI2 gene and CKI2 cDNA from the cki2 mutant, it was found that the CKI2 gene codes for a protein homologous with the plant histidine protein kinase of a two-component system. Particularly high homology was found in two functional domains of the histidine protein kinase, in the histidine kinase domain and the receiver domain.

The cki2 mutant phenotype is similar to the publicly known cki1 mutant in the aspect of forming adventitious shoots in the absence of cytokinins, but the overall structures and amino acid sequences of CKI2 and CKI1 proteins are homologous only on a general level for histidine kinases with different functions, and CKI2 and CKI1 do not exhibit particularly high homology.

The predicted protein also differs considerably in terms of its overall structure and intracellular localization.

When a portion of CKI2 cDNA was introduced into *Arabidopsis thaliana* callus and overexpressed, the transformants exhibited active cell growth and adventitious shoot formation independently of cytokinins in the medium, as was also predicted from the cki2 mutant phenotype. This demonstrated that the CKI2 gene codes for a novel histidine protein kinase involved in cytokinin signal transduction.

The gene of the invention includes any one coding for the amino acid sequence listed as SEQ. ID. No.2, for example. However, it is known that a protein having an amino acid sequence modified with a plurality of amino acid additions, deletions and/or substitutions with other amino acids may maintain similar activity as the original protein. Therefore, the invention also encompasses modified genes coding for proteins having the amino acid sequence listed as SEQ. ID. No.2 modified with one or more amino acid additions, deletions and/or substitutions with other amino acids and having activity involved in cytokinin signal transduction, particularly histidine protein kinase activity.

The degree of modification referred to here is a degree which can be achieved by technical means well known prior to filing of the present application, such as site-specific mutagenesis or PCR. The number of amino acids to be modified while maintaining histidine protein kinase activity may be, for example, no greater than 100, no greater than 50, preferably no greater than 25, and especially no greater than 10.

The invention further provides a gene which can hybridize with nucleic acid, such as DNA, having the nucleotide sequence listed as SEQ. ID. No.1, or a portion thereof, under stringent conditions and which is composed of DNA coding for a protein with histidine protein kinase activity. The stringent conditions referred to here are, for example, conditions of hybridization at 5xSSC, 50°C. The suitable hybridization temperature will depend on the nucleotide sequence and the nucleotide sequence length, and may be selected as appropriate.

The portion of nucleic acid referred to above is a portion coding for at least a few contiguous amino acid sequences, and preferably a portion coding for a few contiguous amino acid sequences in a histidine kinase domain or receiver domain. There may be used, for example, a sequence known to be a well-conserved sequence (Albright et al., Annu. Rev. Genet. 23, p.311-336, 1989; Mizuno, J. Biochem. 123, p.555-563, 1998). More preferably, it refers to a portion or fragment including all or part of a histidine kinase domain or receiver domain of the sequence listed as SEQ. ID. No.1, and having at least 25%, preferably at least 50% and more preferably at least 75% of the total coding sequence listed as SEQ. ID. No.1.

The gene source used as the object of the hybridization may be a cDNA library or genome DNA library prepared from a plant or microorganism with a protein involved in cytokinin signal transduction, particularly a histidine protein kinase, with examples of such plants including *Arabidopsis thaliana,* maize, poplar, petunia, tobacco, rice, tomato, eucalyptus and the like.

The nucleotide sequence of the gene coding for the protein involved in cytokinin signal transduction obtained in this manner has at least 50% or at least 60%, preferably at least 70% or at least 80% and especially at least 90% homology with the nucleotide sequence listed as SEQ. ID. No.1.

The gene coding for a protein with the amino acid sequence listed as SEQ. ID. No.2 may be obtained from *Arabidopsis thaliana* in the form of cDNA or genomic DNA.

A gene with the naturally occurring nucleotide sequence may be obtained by screening of a cDNA library, for example, as explained in detail in the examples. Also, DNA coding for a protein with a modified amino acid sequence may be synthesized by commonly used site-specific mutagenesis or PCR, based on DNA having the naturally occurring nucleotide sequence. For example, a DNA fragment in which the modification is to be introduced may be obtained by restriction enzyme treatment of the naturally occurring cDNA or genomic DNA, and used as a template for site-specific mutagenesis or PCR using primers including the desired mutation, to obtain a DNA fragment with the desired modification introduced therein. The mutation-introduced DNA fragment may then be linked with a DNA fragment coding for another part of the target protein.

Alternatively, when DNA coding for an amino acid sequence longer than the target amino acid sequence, such as the full length amino acid sequence, is digested with a selected restriction enzyme in order to obtain DNA coding for a protein comprising a shortened amino acid sequence, and the obtained DNA fragment consequently does not code for the entire amino acid sequence, a DNA fragment comprising the missing sequence portion may be synthesized and linked thereto.

By using a gene expression system to express the obtained gene in *E. coli* or yeast, it is possible to obtain a histidine protein kinase as the gene product. Alternatively, a histidine protein kinase may also be obtained using antibodies for a protein having the amino acid sequence listed as SEQ. ID. No.2, and such antibodies may be used to clone a histidine protein kinase of another organism.

Thus, the present invention also relates to a recombinant vector, and particularly an expression vector, containing the aforementioned gene, and to a host transformed by the vector. The host used may be a prokaryotic or eukaryotic organism. As prokaryotic organisms there may be mentioned commonly used host microorganisms including bacteria, for example, bacteria belonging to the genus *Escherichia* such as *Escherichia coli*, and microorganisms belonging to the genus *Bacillus* such as *Bacillus subtilis.*

As eukaryotic hosts there may be used lower eukaryotes, for example, eukaryotic microorganisms such as yeast and fungi. As yeasts there may be mentioned, for example, microorganisms belonging to the genus *Saccharomyces,* such as *Saccharomyces cerevisiae,* and as fungi there may be mentioned microorganisms belonging to the genus *Aspergillus,* such as *Aspergillus oryzae* and *Aspergillus niger,* and microorganisms belonging to the genus *Penicillium*. Animal cells or plant cells may also be used, and animal cells which may be used include mouse, hamster, monkey and human cell systems. Plant cells which may be used include cultured cells of tobacco, and cultured cells of *Populus, Eucalyptus* and *Acacia.*

Insect cells such as silkworm cells, or even the silkworm imago itself, may be used as the host. Specifically, there may be used insect cells such as *Spodoptera frugiperda* cells, *Trichoplusia ni* cells or *Bombyx mori* cells and animals cells such as human cells, monkey cells or mouse cells, and more specifically COS cells, Vero cells, CHO cells, L cells, C127 cells, BALB/c 3T3 cells, Sp-2/0 cells and the like.

The expression vector used may be plasmid, phage, phagemid, virus (baculovirus (insect) or vaccinia (animal cells), or the like.

The expression vector of the invention will contain expression control regions, such as a promoter and terminator, replication origin, etc. depending on the type of host into which it is to be introduced. The promoter used for a bacterial expression vector may be a common promoter such as trc promoter, *tac* promoter, *lac* promoter or the like, the promoter used for yeast may be, for example, glyceraldehyde 3-phosphate dehydrogenase promoter, PH05 promoter, adhI promoter, pqk promoter or the like, and the promoter used for fungi may be, for example, amylase promoter, trpC promoter or the like.

As promoters for insects there may be mentioned baculovirus polyhedrin promoter, and as promoters for animal cells there may be mentioned Simian Virus 40 early and late promoters, CMV promoter, HSV-TK promoter or SRα promoter or the like. As promoters for plants there may be mentioned cauliflower mosaic virus 35S promoter and nopaline synthase promoter, and as induction promoters there may be mentioned glutathione-S-transferase II gene promoter, hsp80 promoter, ribulose 2-phosphate carboxylase small subunit gene promoter, and the like.

The expression vector is preferably in a form including not only the above, but also an enhancer, splicing signal, polyA addition signal, selectable marker (for example, a dihydrofolate reductase gene (methotrexate resistance), neo gene (G418 resistance), etc.) and the like. when an enhancer is used, for example, an SV40 enhancer, it may be inserted either upstream or downstream from the gene.

The transformation of the host by the expression vector may be accomplished by an ordinary method well known to those skilled in the art, and such methods are described, for example, in Current Protocols in Molecular Biology, John Wiley & Sons, 1995. The transformants may also be cultured by an ordinary method. Purification of the protein involved in cytokinin signal transduction from the cultured product may be accomplished according to ordinary methods for isolation and purification of proteins, such as ultrafiltration, various column chromatography methods (chromatography using Sepharose, etc.), and the like.

With the current level of technology, the cDNA or gene may be linked under the control of a constitutive or inductible promoter, and the gene may be introduced into and expressed in a plant by a system employing *Agrobacterium* or a particle gun, electroporation or the like, to promote adventitious shoot formation in plants such as roses which are resistant to individual regeneration even by artificial regulation with plant hormones. In addition, by controlling expression of the CKI2 gene it is possible to regulate various physiological effects exhibited by cytokinins in plants, such as lateral bud growth, retardation of aging, flower formation, promotion of fruit enlargement and prevention of fruit abscission.

### Examples

The invention will now be explained in greater detail by way of examples. Unless otherwise specified, the molecular biological methods used were based on Molecular Cloning (Sambrook et al., 1989).

### Example 1 Screening of cytokinin-responding mutants

Activation tagging was carried out using *Arabidopsis thaliana wassileuskija* (WS ecotype) in order to increase transcription of genes involved in the cytokinin synthesis or signal transduction pathway and obtain mutants exhibiting a cytokinin response even in the absence of cytokinins.

The activation tagging vector pPCVICEn4HPT (Hayashi et al., Science, 258, p.1350-1353, 1992) was used for transformation of over 50,000 *Arabidopsis thaliana* WS ecotype calli according to the method of Akama et al. (Akama et al., Plant Cell Rep., 12, 7-11, 1992). pPCVICEn4HPT contains a strong enhancer sequence derived from the cauliflower mosaic virus 35S RNA gene promoter, and after insertion into the plant genome, transcription of the gene adjacent to the enhancer sequence is activated.

The transformed calli were cultured on medium containing no plant hormones. Cell growth of the wild-type *Arabidopsis thaliana* calli is inhibited on medium containing no cytokinins such that adventitious shoots cannot be formed, but adventitious shoots are formed in the presence of cytokinins. Among the transformants, however, there were obtained calli which exhibited a typical cytokinin response and formed adventitious shoots even though no cytokinins were present.

Among these, 4 lines designated as cki1-1, cki1-2, cki1-3 and cki1-4 resulted from overexpression of the same gene, and the causative gene CKI1 has already been reported by the present inventor (Kakimoto, Science, 274:982-985, 1996). In addition to these 4 cki1 lines, there was also obtained a phenotype similar to cki1, i.e. the mutant cki2 having a different causative gene than the causative gene for cki1, while also clearly exhibiting more abundant cell growth than the wild-type callus and exhibiting adventitious shoot formation, in the absence of cytokinins.

### Example 2 Isolation of causative gene of cki2 mutant

Genomic DNA was purified from the cki2 mutant, and after digestion with restriction enzyme SphI, T4 DNA ligase was used for self circularization. This was introduced into *E. coli* and the plasmid was purified from the ampicillin-resistant colonies. The plasmid contains the T-DNA and the DNA sequence adjacent to the T-DNA right border (RB) in the genome of the cki2 mutant.

Oligotex dT-30 (Nihon Roche) was used to purify mRNA from RNA extracted from the cki2 mutant and the entire plant body of a wild-type *Arabidopsis thaliana* WS ecotype, and this was used as the template to create a cDNA library using a Lambda ZAPII cDNA library synthesis kit (Stratagene Co.) by the method recommended by Stratagene Co. The cki2 mutant and wild-type *Arabidopsis thaliana* WS ecotype cDNA libraries were screened using as a probe the genomic sequence adjacent to the RB of the cki2 mutant obtained as described above.

The nucleotide sequence of the CKI2 cDNA obtained from the cDNA library of the wild-type *Arabidopsis thaliana* WS ecotype was determined, and is listed as SEQ. ID. No.1 of the Sequence Listing. The amino acid sequence encoded thereby is listed as SEQ. ID. No.2. The DNA codes for an amino acid sequence homologous with that of a two-component system histidine protein kinase, and particularly it was shown to have high homology with histidine kinase domains and receiver domains which are the two histidine protein kinase functional domains. For example, identical amino acid residues constitute 35% in the histidine kinase domain and 25% in the receiver domain of the sensor histidine kinase ETR1 which is believed to be an ethylene receptor, 41% in the histidine kinase domain and 26% in the receiver domain of the sensor histidine kinase LemA of the prokaryote *Pseudomonas,* and 32% in the histidine kinase domain and 26% in the receiver domain of CKI1 reported by the present inventor.

The amino acid residues that are well conserved among most two-component system factors were also well conserved in CKI2. However, the sequence of approximately 360 amino acids at the N-terminus of CKI2 protein has not been found to have significant homology with any known sequence. Moreover, although a membrane-spanning domain is present at the N-terminus of the sensor histidine kinases ETR1 and CKI1, the hydrophobic regions characteristic of membrane-spanning domains were not found at the N-terminus of CKI2 protein. The CKI2 gene product is therefore predicted to reside in the cytoplasm.

As a result of determining the nucleotide sequence of the cDNA obtained from the cki2 mutant cDNA library and comparing it with the nucleotide sequence of the CKI2 cDNA obtained from the aforementioned wild-type cDNA library, it was shown that in the cki2 mutant, T-DNA was inserted between bases 249 and 250 from the 5' end of the coding region of CKI2 (counting the A of ATG of the translation initiation codon as base 1), and that transcription had begun from within the enhancer sequence on the T-DNA. This indicated that a transcription product of the CKI2 gene lacking the 5' end had been produced in the cki2 mutant, and a CKI2 protein lacking the N-terminus had thus been synthesized. It was surmised that the cki2 mutant exhibited a cytokinin response even in the absence of cytokinins because of constitutive production of the N-terminus lacking CKI2 protein due to the enhancer sequence.

The genomic sequence of the CKI2 gene of *Arabidopsis thaliana* WS ecotype was determined in the following manner. First, total genomic DNA of *Arabidopsis thaliana* WS ecotype was used as a template for amplification by PCR (using LA Taq (Takara), with reaction conditions of 1 minute at 96°C followed by 1 minute at 96°C, 1 minute at 52°C and 3 minutes at 68°C repeated for 4 cycles, and then 1 minute at 96°C, 1 minute at 57°C and 3 minutes at 68°C repeated for 26 cycles, and finally 10 minutes of reaction at 68°C) using primer No.163 (5'-CGCGGATCCACCATGGTCTGTGAAATGGAGAC-3') (SEQ. ID. No.7) and primer No.154 (5'-CCGCTCGAGTCAGTGCAAATACTGTTGCAAAC-3') (SEQ. ID. No.8), and the nucleotide sequence of the coding region of the amplified CKI2 gene was determined.

Genomic DNA of the cki2 mutant was digested with BamHI, and T4-DNA ligase was used for self circularization. This was introduced into *E. coli* DH10B, and the plasmid was purified from *E. coli* of the colonies which exhibited ampicillin resistance. The plasmid contained a DNA sequence adjacent to the T-DNA left border (LB) in the genome of the cki2 mutant. The DNA adjacent to the LB also contained a sequence believed to include the coding region upstream from the T-DNA insertion point and the promoter further upstream from it, of the CKI2 gene. A portion of the nucleotide sequence of this plasmid was determined, and by combining it with the CKI2 cDNA sequence listed as SEQ. ID. No.1, the genomic sequence including the CKI2 coding region and its upstream sequence was determined. This is listed as SEQ. ID. No.3. The amino acid sequence encoded thereby is listed as SEQ. ID. No.4.

PCR was then conducted (using LA Taq (Takara), with 1 minute at 94°C, 30 seconds at 94°C, 30 seconds at 52°C and 3 minutes at 68°C for 30 cycles, and then 7 minutes reaction at 68°C) with *Arabidopsis thaliana Landsberg erecta* (Ler) strain, using primer No.163 and primer No.177 (5'-GGGGTACCTCAGTGCAAATACTGTTGCAAAC-3') (SEQ. ID. No.9), and the nucleotide sequence of the amplified DNA was determined. The sequence was compared with the nucleotide sequence of the CKI2 coding region of WS ecotype and found to be 99.6% identical thereto, and the sequence was therefore designated as the CKI2 gene sequence for Ler strain.

The nucleotide sequence of the CKI2 genome gene of *Arabidopsis thaliana* Ler strain is listed as SEQ. ID. No.5. The protein amino acid sequences deduced from the expected exon sequences of *Arabidopsis thaliana* Ler strain and WS ecotype differed in three amino acid residues. The amino acid sequence encoded thereby is listed as SEQ. ID. No.6.

### Example 3 Overexpression of CKI2 gene

### i) Enhancement of transforming binary vector

pBI121 (Jefferson, R.A. et al., EMBO J., 6:3901-3907, 1987), which is widely used as a binary vector for plant transformation, has the β-glucuronidase (GUS) gene positioned between the cauliflower mosaic virus 35S RNA gene promoter (35S promoter) and the nopaline synthase (NOS) gene terminator in the T-DNA region.

Also, pBE2113-GUS (Mitsuhara, I. et al., Plant Cell Physiol., 37:49-59, 1996) is based on pBI121 and has an added enhancer sequence for reinforced promoter activity and an added omega translation enhancing sequence.

In order to use pBI121 or pBE2113-GUS for overexpression of the specific gene in plants, two types of restriction enzymes are used to remove the GUS gene, and the specific gene is cloned instead at that section. However, since of the enzymes used to remove the GUS gene, only SacI can be used as the enzyme for digesting of the 3' end of the GUS gene in both pBI121 and pBE2113GUS, it was decided to insert a new cloning site.

The NOS gene terminator sequence is present at two locations in both pBI121 and pBE2113-GUS, and the presence of the same sequence in one vector entails the risk of lower stability of the T-DNA in *Agrobacterium* or in the transformed plant. The GUS gene and the NOS gene terminator sequence downstream therefrom were therefore removed from each of the vectors, and the cauliflower mosaic virus 35S RNA gene terminator (35S terminator) was inserted in their place.

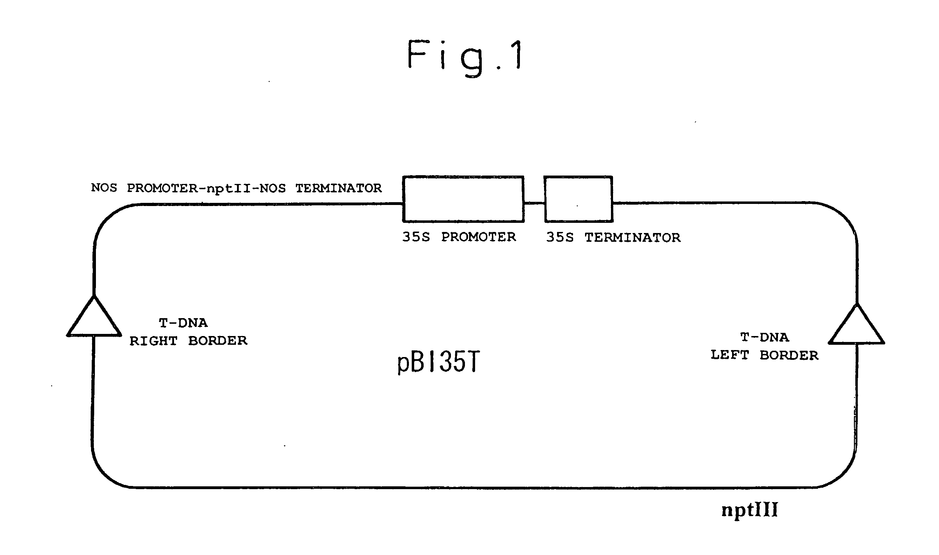

Plasmid pl'barbi (Mengiste, T., Plant J., 12:945-948, 1997) was used as a template for amplification (using LA Taq (Takara), with 1 minute at 94°C, 30 seconds at 94°C, 30 seconds at 52°C and 30 seconds at 68°C for 15 cycles, and then 3 minutes reaction at 68°C) using primer No.271 (5'-TACCCGGGGGTACCGTCGACCTGCAGGCATGCC-3') (SEQ. ID. No.10) and primer No.272 (5'-AAACGACGGCCAGTGAATTCGAGTTCGGCACC-3') (SEQ. ID. No.11) as the primers, the amplified DNA (containing the 35S terminator) was treated with SmaI and EcoRI and the resulting DNA fragment was ligated with a DNA fragment obtained from pBI121 by treatment with SmaI and EcoRI to remove the GUS gene and NOS gene terminator, and the product was designated as pBI35T (Fig. 1).

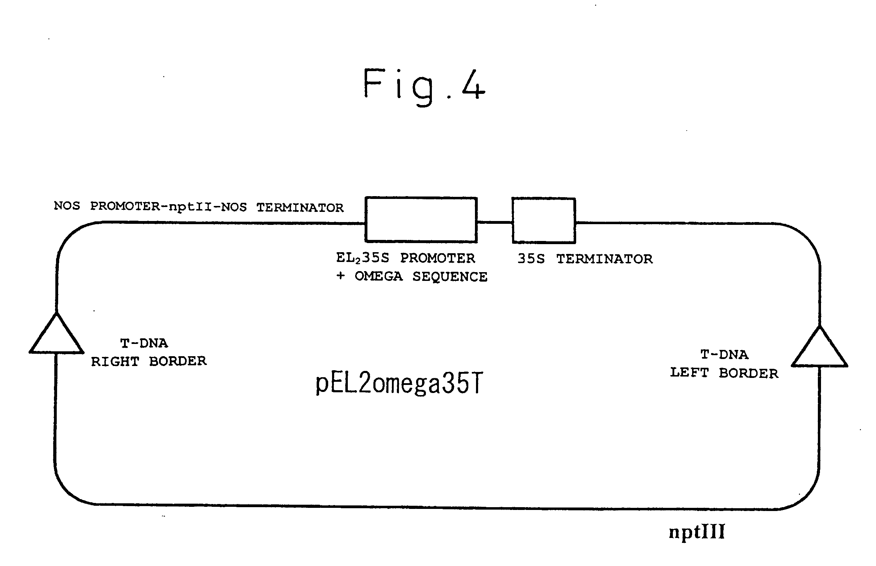

In pBI35T, the unique sites of the multicloning sites between the 35S promoter and 35S terminator were XbaI, BamHI, KpnI and SalI from the end near the 35S promoter. Similarly, the DNA fragment obtained by SmaI and EcoRI treatment of DNA (containing the 35S terminator) amplified using primer No.271 and primer No.272 as the primers with pl'barbi as the template was ligated with a pBE2113-GUS derived DNA fragment obtained from pBE2113-GUS by treatment with SmaI and EcoRI to remove the GUS gene and NOS gene terminator, to obtain pEL2omega35T (Fig. 4). The unique sites of the multicloning sites between the reinforced 35S promoter and 35S terminator in pEL2omega35T were XbaI, BamHI, KpnI and SalI from the end near the 35S promoter.

### ii) Construction of plasmid for CKI2 gene expression

Because growth of *E. coli* containing CKI2 cDNA was poor, cloning downstream from the 35S promoter was expected to be difficult. It was therefore decided to use CKI2 genomic DNA for the experiment of overexpression of all or part of the CKI2 gene in plants. An *Arabidopsis thaliana* Ler strain genome library (Fuse, T. et al., Plant J. 7:849-856, 1995) constructed in Lambda TI2 vector was screened and a clone (LambdaTi.CKI2-2) containing the CKI2 gene was isolated.

The sequence of the CKI2 coding region of the clone is consistent with SEQ. ID. No.5. LambdaTi.CKI2-2 was used as the template for amplification by PCR (using LA Taq (Takara), with reaction for 1 minute at 94°C, followed by 30 seconds at 94°C, 30 seconds at 50°C and 3 minutes at 68°C for 13 cycles, and then 7 minutes reaction at 68°C) with primer No.163 and primer No.177, and cloning was carried out at the restriction enzyme SrfI recognition site of pCR-ScriptSK(+) with blunt ends, to obtain plasmid pCKI2.3LD1. The nucleotide sequence of the CKI2 gene portion of pCKI2.3LD1 matched the nucleotide sequence of the *Arabidopsis thaliana* Ler strain CKI2 gene.

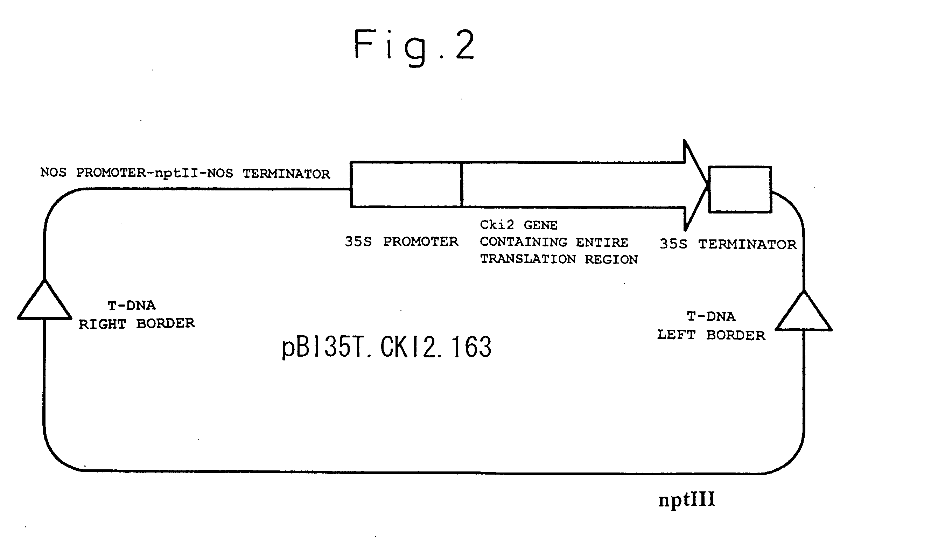

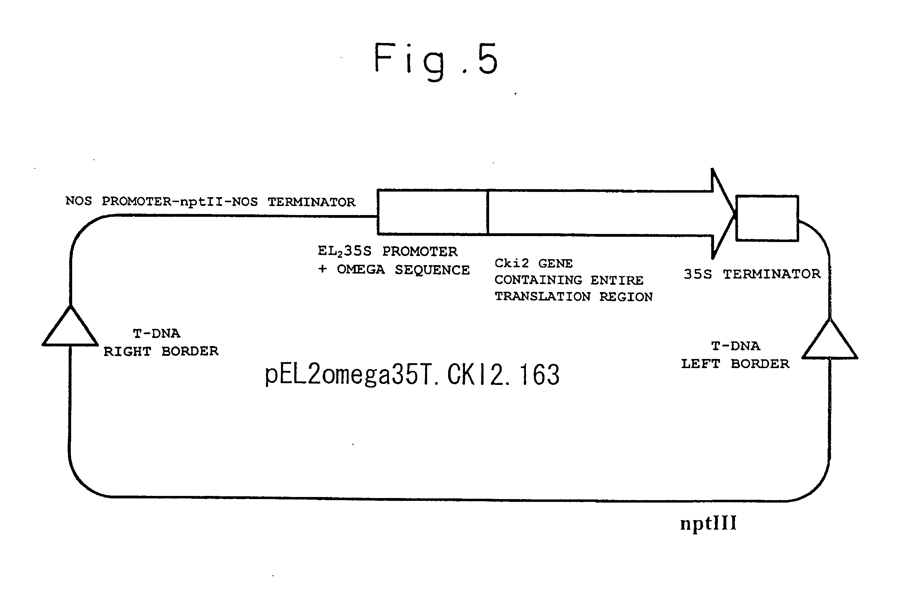

After digesting pCKI2.3LD1 with KpnI and BamHI, the fragment containing the CKI2 gene was cloned between the KpnI site and BamHI site of pBI35T and between the KpnI site and BamHI site of pEL2omega35T, and these were designated as pBI35T.CKI2.163 (Fig. 2) and pEL20mega35T.CKI2.163 (Fig. 5), respectively. These two clones had the entire coding region of the CKI2 gene cloned in the sense direction with respect to the 35S promoter.

Also, LambdaTi.CKI2-2 was used as the template for amplification by PCR (using LA Taq (Takara), with reaction for 1 minute at 94°C, followed by 30 seconds at 94°C, 30 seconds at 50°C and 3 minutes at 68°C for 13 cycles, and then 7 minutes reaction at 68°C) with primer No.162 (5'-CGCGGATCCACCATGCTTGAGGCGAGTACTG-3') (SEQ. ID. No.12) and primer No.177, and cloning was carried out at the restriction enzyme SrfI recognition site of pCR-ScriptSK(+) with blunt ends, to obtain plasmid pCKI2.2L16. The nucleotide sequence of the CKI2 gene portion of pCKI2.2L16 matched the nucleotide sequence of the *Arabidopsis thaliana* Ler strain CKI2 gene.

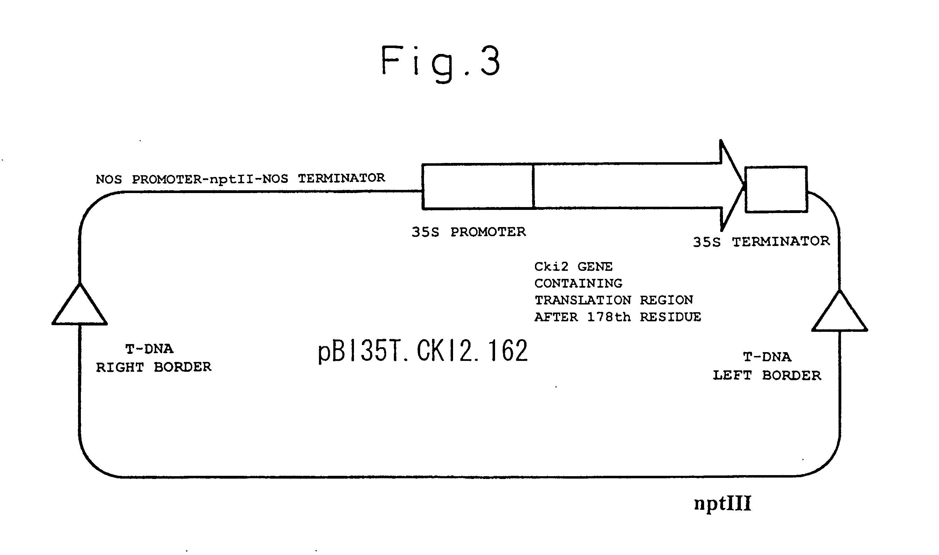

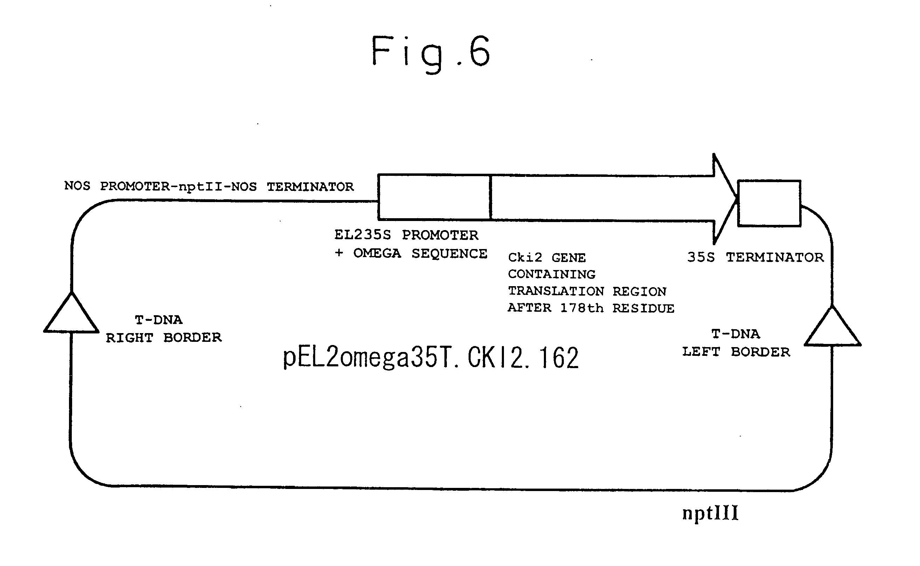

After digesting pCKI2.2L16 with KpnI and BamHI, the fragment containing a part of the CKI2 gene was cloned between the BamHI site and KpnI site of pBI35T and between the BamHI site and KpnI site of pEL2omega35T, and these were designated as pBI35T.CKI2.162 (Fig. 3) and pEL2omega35T.CKI2.162 (Fig. 6), respectively. These two clones coded for the portion from the 178th methionine to the carboxyl end of the protein encoded by the CKI2 gene.

The following experiment was conducted to investigate whether or not a cytokinin response is elicited by overexpression of the CKI2 gene as the entire region of the CKI2 protein or as the region after the 178th methionine. *Arabidopsis thaliana* WS ecotype calli were transformed by coculturing with *Agrobacteria* containing pBI35T, pBI35T.CKI2.163, pBI35T.CKI2.162, pEL2omega35T, pEL20mega35T.CKI2.163 or pEL20mega35T.CKI2.162, and were cultured on the following three different solid medium, that is, GM medium (4.32 g/l Murashige-Skoog salts (Sigma), 1% sucrose, 10 ml/l 5% 2-(N-morpholino)ethanesulfonic acid (adjusted to pH 5.8 with KOH), 100 mg/l inositol, 10 mg/l thiamin-HCl, 1 mg/l pyridoxine-HCl, 1 mg/l nicotinic acid and 0.3% Phytage ITM (Sigma)) with addition of solid medium Z-0 containing 50 mg/l kanamycin sulfate, 100 mg/l cefotaxime, 100 mg/l vancomyin and 0.3 mg/l indoleacetic acid, and further addition of solid medium Z-0.1 containing 0.1 mg/l transzeatin and solid medium Z-1 containing 1 mg/l transzeatin.

The calli transformed with pBI35T and the calli transformed with pEL2omega35T exhibited no virescence and formed no shoots (buds) on the Z-0 medium, but exhibited virescence and shoot (bud) formation with low frequency on Z-0.1 medium, whereas virescence and shoot formation were found in over half of the calli on the Z-1 medium. In contrast, almost all of the calli transformed with pBI35T.CKI2.163, pBI35T.CKI2.162, pEL2omega35T.CKI2.163 and pEL2omega35T.CKI2.162 exhibited virescence and shoot formation on all of the medium, Z-0, Z-0.1 and Z-1.

All of the calli transformed with these vectors exhibited the most rapid shoot formation on Z-0 and Z-0.1 medium, which tended to be more rapid shoot formation than the calli transformed with the control vectors pBI35T and pEL2omega35T, with culturing at any cytokinin concentration.

These results indicate that a cytokinin response is elicited in the absence of cytokinins with overexpression of either a portion or the entire region of the CKI2 gene.

### Example 4 Expression of CKI2 gene in plant bodies

Next, the effect of introducing the CKI2 gene on plant individuals was investigated. Preparation of the constructs for introduction into the plants was carried out in the same manner as Example 3.

Specifically, the CKI2 cDNA obtained in Example 2 was used as the template for amplification of the region corresponding from the 178th methionine to the stop codon of the CKI2 protein by PCR (Stratagene) using primer No.162 and primer No.177, and this was inserted downstream from the cauliflower mosaic virus 35S promoter of the binary vector.

After confirming the nucleotide sequences of the prepared constructs, all of them included mutations and therefore the regions containing the mutated nucleotides were removed by digestion with restriction enzymes ScaI and SacI, and the mutations were repaired by ligation using the corresponding ScaI and SacI restriction enzyme fragments from the genomic clone obtained in Example 2. The clone prepared in this manner was designated as pTK031.

Next, the vacuum infiltration method (Araki, T., Sokubutsu Saibou Kogaku [Plant Cell Engineering] Series 4, Model Plant Experiment Protocols, p.109-113, Shujunsha Publishing) was used for introduction of pTK031 or a binary vector with no insert into *Arabidopsis thaliana,* and the obtained seeds were disseminated in agar medium containing kanamycin as a selectable marker for the transformants.

The medium was incubated for 12 days at 22°C under light condition and observed. At this stage, none of the 19 calli transformed with the vector alone formed scapes, but scape elongation was found in 11 of the 45 calli transformed with pTK031.

In the case of *Arabidopsis thaliana,* scape elongation is accompanied by Horal meristem formation, and therefore these results demonstrated that introduction of pTK031 hastened the period of shifting from vegetative growth to reproductive growth.

It was thus shown that in transformants in which a portion of the CKI2 gene is cloned under the cauliflower mosaic virus 35S promoter, transition from vegetative growth to reproductive growth occurs much more rapidly. Consequently, by using all or a portion of the CKI2 gene it is possible to artificially regulate the Horal formation period.

### Industrial Applicability

In the manner described above, activation tagging was carried out to isolate the CKI2 gene coding for a histidine protein kinase involved in the cytokinin signal transduction pathway from *Arabidopsis thaliana*. In cki2 mutants, a cytokinin response including cell division and adventitious shoot formation was exhibited even in the absence of cytokinins due to constitutive synthesis of CKI2 protein lacking the N-terminal region. It is therefore clear that the cytokinin response can be controlled by controlling expression of the CKI2 gene, and thus by controlling expression of the CKI2 gene, it has become possible to control the various physiological effects exhibited by cytokinins in plants.

## Claims

1. A gene coding for a protein having the amino acid sequence listed as SEQ. ID. No.2, and involved in cytokinin signal transduction.

2. A gene coding for a protein having the amino acid sequence listed as SEQ. ID. No.2 modified with one or more amino acid additions, deletions and/or substitutions with other amino acids, and involved in cytokinin signal transduction.

3. A gene which hybridizes with nucleic acid having the nucleotide sequence listed as SEQ. ID. No.1, or a portion thereof, under stringent conditions and which codes for a protein involved in cytokinin signal transduction.

4. A gene according to any one of claims 1 to 3, wherein said protein is a histidine protein kinase.

5. A vector containing a gene according to any one of claims 1 to 4.

6. A host transformed by a vector according to claim 5.

7. A protein encoded by a gene according to any one of claims 1 to 4.

8. A method of producing a protein with histidine protein kinase activity, **characterized in that** a host according to claim 6 is cultured or cultivated and said protein is obtained from said host.

9. A plant or plant cells having introduced therein a gene according to any one of claims 1 to 4.

10. A method of regulating growth of a plant or plant cells, whereby a gene according to any one of claims 1 to 4 is introduced into a plant or plant cells and said gene is expressed.

11. A method of inducing adventitious shoot formation in a plant or plant cells, whereby a gene according to any one of claims 1 to 4 is introduced into a plant or plant cells and said gene is expressed.

12. A method of regulating the Horal formation period of a plant, whereby a gene according to any one of claims 1 to 4 is introduced into a plant or plant cells and said gene is expressed.


SEQUENCE LISTING
<110>     Suntory Limited
<110>     Nippon Paper Industries
<120>     Gene coding for protein participating in signal
transduction of cytokinin
<130>     993776
<160>     2
<210>     1
<211>     3096
<212>     DNA
<213>     Arabidopsis thaliana
<221>     CDS
<222>     (187)...(2952)
<223>     Nucleotide sequence coding for histidine protein
kinase
<400>     1
gactcttctt cagatctact cactccttct ttctctcctt cttcttcttc atttttccgg   60
tgaccggagt cggagaaggt tctttattca gatcaaggtt ctggcttaaa gaaaaaagtt  120
gtttgaattt tgagatttgt ctggtccatt gtgttgctgt tgttgtatga agagaaacct  180
tgatca atg gtc tgt gaa atg gag act gat cag att gag gaa atg gat     228
       Met Val Cys Glu Met Glu Thr Asp Gln Ile Glu Glu Met Asp
        1               5                   10
gtc gaa gtt ttg tct tcg atg tgg ccc gaa gat gtt gga act gaa gct    276
Val Glu Val Leu Ser Ser Met Trp Pro Glu Asp Val Gly Thr Glu Ala
 15                  20                  25                  30
gac aaa cag ttc aac gtc gag aaa cct gcc gga gat tta gac acg ttg    324
Asp Lys Gln Phe Asn Val Glu Lys Pro Ala Gly Asp Leu Asp Thr Leu
                 35                  40                  45
aaa gaa gtt act atc gag aca cgg acc att gcg gat atg aca cgg tta    372
Lys Glu Val Thr Ile Glu Thr Arg Thr Ile Ala Asp Met Thr Arg Leu
             50                  55                  60
cca aac cta ttg aat tcg act cat caa ggc tcc tct caa cta acc aac    420
Pro Asn Leu Leu Asn Ser Thr His Gln Gly Ser Ser Gln Leu Thr Asn
         65                  70                  75
ctt gtg aaa caa tgg gag tat atg caa gac aac gcg gtt cgg ctg tta    468
Leu Val Lys Gln Trp Glu Tyr Met Gln Asp Asn Ala Val Arg Leu Leu
     80                  85                  90

aaa gaa gag cta aaa aat ctc gat aga cag aga gaa gaa gcc gag gct    516
Lys Glu Glu Leu Lys Asn Leu Asp Arg Gln Arg Glu Glu Ala Glu Ala
 95                 100                 105                 110
aaa gag ttg aag atc att gag gag tat aag ttt gag agc aac gag cct    564
Lys Glu Leu Lys Ile Ile Glu Glu Tyr Lys Phe Glu Ser Asn Glu Pro
                115                 120                 125
gag aat gtt ccg gtt ttg gat gag acg agt gat ttg ttc cgc agg ttt    612
Glu Asn Val Pro Val Leu Asp Glu Thr Ser Asp Leu Phe Arg Arg Phe
            130                 135                 140
agg cag aaa aaa cga gat gcc ttg gtc gat agc aag aag att gag atc    660
Arg Gln Lys Lys Arg Asp Ala Leu Val Asp Ser Lys Lys Ile Glu Ile
        145                 150                 155
tat gag gag ttt gac act gtt gca tat tgg aaa cag aag gcg ttg agt    708
Tyr Glu Glu Phe Asp Thr Val Ala Tyr Trp Lys Gln Lys Ala Leu Ser
    160                 165                 170
ctt gag aaa atg ctt gag gcg agt act gag aga gaa agg cga ttg atg    756
Leu Glu Lys Met Leu Glu Ala Ser Thr Glu Arg Glu Arg Arg Leu Met
170                 180                 185                 190
gag aag ctg agt gag agt ttg aaa act atg gag agt cag tca gca ccg    804
Glu Lys Leu Ser Glu Ser Leu Lys Thr Met Glu Ser Gln Ser Ala Pro
                195                 200                 205
gtc caa gag ctt act cag aat ctt aag aga gct gaa ggt ttc ttg cat    852
Val Gln Glu Leu Thr Gln Asn Leu Lys Arg Ala Glu Gly Phe Leu His
            210                 215                 220
ttc ata ctt cag aat gca cct att gtt atg ggc cat cag gat aaa gat    900
Phe Ile Leu Gln Asn Ala Pro Ile Val Met Gly His Gln Asp Lys Asp
        225                 230                 235
tta cgc tac ttg ttc atc tac aac aag tat cct agt tta cgg gaa cag    948
Leu Arg Tyr Leu Phe Ile Tyr Asn Lys Tyr Pro Ser Leu Arg Glu Gln
    240                 245                 250
gac att ttg gga aaa aca gac gtg gag ata ttc cat gga ggt gga gtt    996
Asp Ile Leu Gly Lys Thr Asp Val Glu Ile Phe His Gly Gly Gly Val
255                 260                 265                 270
aaa gaa tct gaa gat ttc aag aga gaa gtt ctt gag aaa gga aaa gct   1044
Lys Glu Ser Glu Asp Phe Lys Arg Glu Val Leu Glu Lys Gly Lys Ala
                275                 280                 285

tca aag aga gag atc aca ttt act aca gat tta ttt gga tca aag acg 1092
Ser Lys Arg Glu Ile Thr Phe Thr Thr Asp Leu Phe Gly Ser Lys Thr
            290                 295                 300
ttt ttg ata tat gtt gag cct gtt tac aac aaa gct ggc gag aaa atc 1140
Phe Leu Ile Tyr Val Glu Pro Val Tyr Asn Lys Ala Gly Glu Lys Ile
        305                 310                 315
ggt ata aac tac atg gga atg gaa gta act gat cag gta gtg aaa agg 1188
Gly Ile Asn Tyr Met Gly Met Glu Val Thr Asp Gln Val Val Lys Arg
    320                 325                 330
gag aaa atg gcg aaa ctt aga gaa gat aac gct gtg aga aag gcg atg 1236
Glu Lys Met Ala Lys Leu Arg Glu Asp Asn Ala Val Arg Lys Ala Met
335                 340                 345                 350
gaa tca gaa ctg aac aag act att cac att aca gag gag aca atg aga 1284
Glu Ser Glu Leu Asn Lys Thr Ile His Ile Thr Glu Glu Thr Met Arg
                355                 360                 365
gca aag cag atg cta gcg acg atg tct cat gag ata agg tca cca ttg 1332
Ala Lys Gln Met Leu Ala Thr Met Ser His Glu Ile Arg Ser Pro Leu
            370                 375                 380
tca gga gta gtg gga atg gct gag ata ctt tca act aca aaa ctg gat 1380
Ser Gly Val Val Gly Met Ala Glu Ile Leu Ser Thr Thr Lys Leu Asp
        385                 390                 395
aaa gag caa aga cag ttg ttg aat gtc atg atc tct tct ggt gat ttg 1428
Lys Glu Gln Arg Gln Leu Leu Asn Val Met Ile Ser Ser Gly Asp Leu
    400                 405                 410
gtg ctt cag cta atc aac gac att ctt gat ctc tcc aag gtt gaa tca 1476
Val Leu Gln Leu Ile Asn Asp Ile Leu Asp Leu Ser Lys Val Glu Ser
415                 420                 425                 430
ggt gtg atg aga tta gaa gct aca aag ttt cga cca aga gaa gta gtg 1524
Gly Val Met Arg Leu Glu Ala Thr Lys Phe Arg Pro Arg Glu Val Val
                435                 440                 445
aag cat gtg cta cag aca gct gca gca tcg ctg aag aaa tct ttg aca 1572
Lys His Val Leu Gln Thr Ala Ala Ala Ser Leu Lys Lys Ser Leu Thr
            450                 455                 460
tta gaa gga aac att gca gat gat gtt cct att gag gta gtt gga gat 1620
Leu Glu Gly Asn Ile Ala Asp Asp Val Pro Ile Glu Val Val Gly Asp
        465                 470                 475

gtt cta agg att agg cag atc ctc acc aat ttg ata agc aat gct atc 1668
Val Leu Arg Ile Arg Gln Ile Leu Thr Asn Leu Ile Ser Asn Ala Ile
    480                 485                 490
aag ttt aca cat gaa gga aat gta gga atc aaa ctc caa gtg ata tca 1716
Lys Phe Thr His Glu Gly Asn Val Gly Ile Lys Leu Gln Val Ile Ser
495                 500                 505                 510
gaa cca tcc ttt gtg cgg gat aac gca ttg aac gca gac acc gag gaa 1764
Glu Pro Ser Phe Val Arg Asp Asn Ala Leu Asn Ala Asp Thr Glu Glu
                515                 520                 525
cac gaa caa aac ggt ttg acc gag act tca gtt tgg att tgc tgt gac 1812
His Glu Gln Asn Gly Leu Thr Glu Thr Ser Val Trp Ile Cys Cys Asp
            530                 535                 540
gta tgg gac act gga att gga atc cca gaa aac gct ctt cca tgt ttg 1860
Val Trp Asp Thr Gly Ile Gly Ile Pro Glu Asn Ala Leu Pro Cys Leu
        545                 550                 555
ttc aag aag tac atg caa gca agc gct gat cat gcc cgg aaa tac ggt 1908
Phe Lys Lys Tyr Met Gln Ala Ser Ala Asp His Ala Arg Lys Tyr Gly
    560                 565                 570
ggg act ggt ctc gga ctt gct att tgt aaa cag ctg gtt gag tta atg 1956
Gly Thr Gly Leu Gly Leu Ala Ile Cys Lys Gln Leu Val Glu Leu Met
575                 580                 585                 590
gga ggc caa ctc act gtg aca agc cgg gtg agc gaa ggt tca acg ttc 2004
Gly Gly Gln Leu Thr Val Thr Ser Arg Val Ser Glu Gly Ser Thr Phe
                595                 600                 605
aca ttt ata tta ccc tac aaa gtt gga aga tca gat gat tat tca gat 2052
Thr Phe Ile Leu Pro Tyr Lys Val Gly Arg Ser Asp Asp Tyr Ser Asp
            610                 615                 620
gat caa gat gag ttc tct gat atg gcg gat caa caa tct gaa cca gac 2100
Asp Gln Asp Glu Phe Ser Asp Met Ala Asp Gln Gln Ser Glu Pro Asp
        625                 630                 635
gat aca gct gaa gga tat ttc cag ttt aaa ccg ctc tta gga tcg ata 2148
Asp Thr Ala Glu Gly Tyr Phe Gln Phe Lys Pro Leu Leu Gly Ser Ile
    640                 645                 650
tat tcg aat ggc gga ccg ggg atc agc aat gac ttc tta cct cat aaa 2196
Tyr Ser Asn Gly Gly Pro Gly Ile Ser Asn Asp Phe Leu Pro His Lys
655                 660                 665                 670

gtc atg ctt act agt cct att aag ctc atc aat ggt ttt gtc gct gat     2244
Val Met Leu Thr Ser Pro Ile Lys Leu Ile Asn Gly Phe Val Ala Asp
                675                 680                 685
ccc tct aat aac act gga cag agc gag atg cta cag ctt gaa aac ggt     2292
Pro Ser Asn Asn Thr Gly Gln Ser Glu Met Leu Gln Leu Glu Asn Gly
            690                 695                 700
ggt tac atg gat gaa tct aaa ctc gaa acc agt tct ggt cat tgc cct     2340
Gly Tyr Met Asp Glu Ser Lys Leu Glu Thr Ser Ser Gly His Cys Pro
        705                 710                 715
gaa tca gct cac caa tat gag aat gga aat ggt cga tgt ttc tct aag     2388
Glu Ser Ala His Gln Tyr Glu Asn Gly Asn Gly Arg Cys Phe Ser Lys
    720                 725                 730
gaa tct gaa tct tgt agc agt tca caa gct agc tca gaa ggt gga acc     2436
Glu Ser Glu Ser Cys Ser Ser Ser Gln Ala Ser Ser Glu Gly Gly Thr
735                 740                 745                 750
tta gaa atg gag tca gag ctc aca gtt tca tct cat agg gaa gag gaa     2484
Leu Glu Met Glu Ser Glu Leu Thr Val Ser Ser His Arg Glu Glu Glu
                755                 760                 765
aaa gcc gag aca gaa gta aaa gaa aca tca aag cca aag att ttg ctt     2532
Lys Ala Glu Thr Glu Val Lys Glu Thr Ser Lys Pro Lys Ile Leu Leu
            770                 775                 780
gtg gaa gat aat aag atc aac atc atg gtt gca aag tcg atg atg aag     2580
Val Glu Asp Asn Lys Ile Asn Ile Met Val Ala Lys Ser Met Met Lys
        785                 790                 795
cag tta ggc cat acc atg gat att gct aat aat gga gtt gaa gcc ata     2628
Gln Leu Gly His Thr Met Asp Ile Ala Asn Asn Gly Val Glu Ala Ile
    800                 805                 810
acc gcg att aat agc tct agc tac gat ctg gta ctc atg gat gtg tgc     2676
Thr Ala Ile Asn Ser Ser Ser Tyr Asp Leu Val Leu Met Asp Val Cys
815                 820                 825                 830
atg ccg gtg ctc gat ggt tta aaa gct aca aga ctg atc cgt tcg tat     2724
Met Pro Val Leu Asp Gly Leu Lys Ala Thr Arg Leu Ile Arg Ser Tyr
                835                 840                 845
gaa gaa act ggg aac tgg aat gct gca ata gaa gcc gga gta gat ata     2772
Glu Glu Thr Gly Asn Trp Asn Ala Ala Ile Glu Ala Gly Val Asp Ile
            850                 855                 860

tcg aca tcg gag aat gaa caa gtt tgt atg cgt ccc aca aac cgg ctg 2820
Ser Thr Ser Glu Asn Glu Gln Val Cys Met Arg Pro Thr Asn Arg Leu
        865                 870                 875
cct ata atc gcg atg acg gca aat act tta gca gag agt tca gaa gaa 2868
Pro Ile Ile Ala Met Thr Ala Asn Thr Leu Ala Glu Ser Ser Glu Glu
    880                 885                 890
tgt tat gca aat ggt atg gac tcg ttt att tcg aaa cct gta acg ttg 2916
Cys Tyr Ala Asn Gly Met Asp Ser Phe Ile Ser Lys Pro Val Thr Leu
895                 900                 905                 910
caa aaa ctg aga gag tgt ttg caa cag tat ttg cac tgagatttca 2962
Gln Lys Leu Arg Glu Cys Leu Gln Gln Tyr Leu His
                915                 920
gatttttgtg ttttgtagat taagaaatgg ttgtgttgta tataaattgt gtaggaaaaa 3022
agttttggag agctactaag tagcttcctc tttttagaga tgtatagttc aataaaaaaa 3082
aaaaaaaaaa aaaa 3096
<210> 2
<211> 922
<212> PRT
<213> Arabidopsis thaliana
<223> Amino acid sequence of histidine protein kinase
<400> 2
Met Val Cys Glu Met Glu Thr Asp Gln Ile Glu Glu Met Asp Val Glu
1               5                   10                  15
Val Leu Ser Ser Met Trp Pro Glu Asp Val Gly Thr Glu Ala Asp Lys
            20                  25                  30
Gln Phe Asn Val Glu Lys Pro Ala Gly Asp Leu Asp Thr Leu Lys Glu
        35                  40                  45
Val Thr Ile Glu Thr Arg Thr Ile Ala Asp Met Thr Arg Leu Pro Asn
    50                  55                  60
Leu Leu Asn Ser Thr His Gln Gly Ser Ser Gln Leu Thr Asn Leu Val
65                  70                  75                  80
Lys Gln Trp Glu Tyr Met Gln Asp Asn Ala Val Arg Leu Leu Lys Glu
                85                  90                  95
Glu Leu Lys Asn Leu Asp Arg Gln Arg Glu Glu Ala Glu Ala Lys Glu
            100                 105                 110
Leu Lys Ile Ile Glu Glu Tyr Lys Phe Glu Ser Asn Glu Pro Glu Asn
        115                 120                 125

Val Pro Val Leu Asp Glu Thr Ser Asp Leu Phe Arg Arg Phe Arg Gln
    130                 135                 140
Lys Lys Arg Asp Ala Leu Val Asp Ser Lys Lys Ile Glu Ile Tyr Glu
145                 150                 155                 160
Glu Phe Asp Thr Val Ala Tyr Trp Lys Gln Lys Ala Leu Ser Leu Glu
                165                 170                 175
Lys Met Leu Glu Ala Ser Thr Glu Arg Glu Arg Arg Leu Met Glu Lys
            180                 185                 190
Leu Ser Glu Ser Leu Lys Thr Met Glu Ser Gln Ser Ala Pro Val Gln
        195                 200                 205
Glu Leu Thr Gln Asn Leu Lys Arg Ala Glu Gly Phe Leu His Phe Ile
    210                 215                 220
Leu Gln Asn Ala Pro Ile Val Met Gly His Gln Asp Lys Asp Leu Arg
225                 230                 235                 240
Tyr Leu Phe Ile Tyr Asn Lys Tyr Pro Ser Leu Arg Glu Gln Asp Ile
                245                 250                 255
Leu Gly Lys Thr Asp Val Glu Ile Phe His Gly Gly Gly Val Lys Glu
            260                 265                 270
Ser Glu Asp Phe Lys Arg Glu Val Leu Glu Lys Gly Lys Ala Ser Lys
        275                 280                 285
Arg Glu Ile Thr Phe Thr Thr Asp Leu Phe Gly Ser Lys Thr Phe Leu
    290                 295                 300
Ile Tyr Val Glu Pro Val Tyr Asn Lys Ala Gly Glu Lys Ile Gly Ile
305                 310                 315                 320
Asn Tyr Met Gly Met Glu Val Thr Asp Gln Val Val Lys Arg Glu Lys
                325                 330                 335
Met Ala Lys Leu Arg Glu Asp Asn Ala Val Arg Lys Ala Met Glu Ser
            340                 345                 350
Glu Leu Asn Lys Thr Ile His Ile Thr Glu Glu Thr Met Arg Ala Lys
        355                 360                 365
Gln Met Leu Ala Thr Met Ser His Glu Ile Arg Ser Pro Leu Ser Gly
    370                 375                 380
Val Val Gly Met Ala Glu Ile Leu Ser Thr Thr Lys Leu Asp Lys Glu
385                 390                 395                 400
Gln Arg Gln Leu Leu Asn Val Met Ile Ser Ser Gly Asp Leu Val Leu
                405                 410                 415
Gln Leu Ile Asn Asp Ile Leu Asp Leu Ser Lys Val Glu Ser Gly Val
            420                 425                 430

Met Arg Leu Glu Ala Thr Lys Phe Arg Pro Arg Glu Val Val Lys His
        435                 440                 445
Val Leu Gln Thr Ala Ala Ala Ser Leu Lys Lys Ser Leu Thr Leu Glu
    450                 455                 460
Gly Asn Ile Ala Asp Asp Val Pro Ile Glu Val Val Gly Asp Val Leu
465                 470                 475                 480
Arg Ile Arg Gln Ile Leu Thr Asn Leu Ile Ser Asn Ala Ile Lys Phe
                485                 490                 495
Thr His Glu Gly Asn Val Gly Ile Lys Leu Gln Val Ile Ser Glu Pro
            500                 505                 510
Ser Phe Val Arg Asp Asn Ala Leu Asn Ala Asp Thr Glu Glu His Glu
        515                 520                 525
Gln Asn Gly Leu Thr Glu Thr Ser Val Trp Ile Cys Cys Asp Val Trp
    530                 535                 540
Asp Thr Gly Ile Gly Ile Pro Glu Asn Ala Leu Pro Cys Leu Phe Lys
545                 550                 555                 560
Lys Tyr Met Gln Ala Ser Ala Asp His Ala Arg Lys Tyr Gly Gly Thr
                565                 570                 575
Gly Leu Gly Leu Ala Ile Cys Lys Gln Leu Val Glu Leu Met Gly Gly
            580                 585                 590
Gln Leu Thr Val Thr Ser Arg Val Ser Glu Gly Ser Thr Phe Thr Phe
        595                 600                 605
Ile Leu Pro Tyr Lys Val Gly Arg Ser Asp Asp Tyr Ser Asp Asp Gln
    610                 615                 620
Asp Glu Phe Ser Asp Met Ala Asp Gln Gln Ser Glu Pro Asp Asp Thr
625                 630                 635                 640
Ala Glu Gly Tyr Phe Gln Phe Lys Pro Leu Leu Gly Ser Ile Tyr Ser
                645                 650                 655
Asn Gly Gly Pro Gly Ile Ser Asn Asp Phe Leu Pro His Lys Val Met
            660                 665                 670
Leu Thr Ser Pro Ile Lys Leu Ile Asn Gly Phe Val Ala Asp Pro Ser
        675                 680                 685
Asn Asn Thr Gly Gln Ser Glu Met Leu Gln Leu Glu Asn Gly Gly Tyr
    690                 695                 700
Met Asp Glu Ser Lys Leu Glu Thr Ser Ser Gly His Cys Pro Glu Ser
705                 710                 715                 720
Ala His Gln Tyr Glu Asn Gly Asn Gly Arg Cys Phe Ser Lys Glu Ser
                725                 730                 735

Glu Ser Cys Ser Ser Ser Gln Ala Ser Ser Glu Gly Gly Thr Leu Glu
            740                 745                 750
Met Glu Ser Glu Leu Thr Val Ser Ser His Arg Glu Glu Glu Lys Ala
        755                 760                 765
Glu Thr Glu Val Lys Glu Thr Ser Lys Pro Lys Ile Leu Leu Val Glu
    770                 775                 780
Asp Asn Lys Ile Asn Ile Met Val Ala Lys Ser Met Met Lys Gln Leu
785                 790                 795                 800
Gly His Thr Met Asp Ile Ala Asn Asn Gly Val Glu Ala Ile Thr Ala
                805                 810                 815
Ile Asn Ser Ser Ser Tyr Asp Leu Val Leu Met Asp Val Cys Met Pro
            820                 825                 830
Val Leu Asp Gly Leu Lys Ala Thr Arg Leu Ile Arg Ser Tyr Glu Glu
        835                 840                 845
Thr Gly Asn Trp Asn Ala Ala Ile Glu Ala Gly Val Asp Ile Ser Thr
    850                 855                 860
Ser Glu Asn Glu Gln Val Cys Met Arg Pro Thr Asn Arg Leu Pro Ile
865                 870                 875                 880
Ile Ala Met Thr Ala Asn Thr Leu Ala Glu Ser Ser Glu Glu Cys Tyr
                885                 890                 895
Ala Asn Gly Met Asp Ser Phe Ile Ser Lys Pro Val Thr Leu Gln Lys
            900                 905                 910
Leu Arg Glu Cys Leu Gln Gln Tyr Leu His
        915                 920
<210> 3
<211> 4679
<212> DNA
<213> Arabidopsis thaliana
<220>
<221> exon 1
<222> (230) ... (307)
<221> exon 2
<222> (865) ... (1677)
<221> exon 3
<222> (1755) ... (1811)
<221> exon 4
<222> (1904) ... (2131)
<221> exon 5

<222>     (2214) ··· (2307)
<221>     exon 6
<222>     (2389) ··· (2595)
<221>     exon 7
<222>     (2697) ··· (2827)
<221>     exon 8
<222>     (2905) ··· (2954)
<221>     exon 9
<222>     (3028) ··· (3209)
<221>     exon 10
<222>     (3306) ··· (3409)
<221>     exon 11
<222>     (3492) ··· (4214)
<221>     exon 12
<222>     (4303) ··· (4467)
<221>     exon 13
<222>     (4557) ··· (4679)
<223>     Nucleotide sequence of genomic DNA coding for hist
idine protein kinase
<400>     3
gagaaagaga gagaagagaa gagaagagaa gagatgaata taatatacat tgacctctct    60
gtctcatgag caagcacatg tcctctctct ctctctctct ctttctcttc tccgtaaaaa   120
aaattaccaa tctttcacaa ttcattcaca tgctcctctc tctttcttca ttggctattc   180
tcttcccaat gctcatttga agtttattta ctctactccc gacgaccacg actcttcttc   240
agatctactc actccttctt tctctccttc ttcttcttca tttttccggt gaccggagtc   300
ggagaaggta aagcttctga tcccttcccc tttcctccgg catactcgtt cctctgcttt   360
gtctttcgtt tttttcattt ctttctgttt cagccttttg catctcgaga cttcatgatt   420
acaatttctt tatgtttctc aatgcaagat tttcgtttat aatatatgat cactgatgtc   480
aagaataaat caccaaagat ttttttcttt ccatatattt tttccgatca tcgctcaaaa   540
aaaagtttca tcgtttaaat ttattttcag attttatcgt ttattggtga attttatgat   600
cctagttgat aattcaatct gaaaaaagaa actgatacag ttttcttttt gaagctctgc   660
aaatttctga tttttaagct cgaatcatat aatttgaagt ttcccgctaa tgttcaatca   720
gaattggtcc acacacttag ttgttgtgga ccctttgcaa aattctattt tagttcatta   780
tttttcacat tttacagatc aagattctct gatggagatg gtttgtcctg actctaacct   840
ctgtgtttga aaatatctat acaggttctt tattcagatc aaggttctgg cttaaagaaa   900
aaagttgttt gaattttgag atttgtctgg tccattgtgt tgctgttgtt gtatgaagag   960
aaaccttgat ca                                                       972

atg gtc tgt gaa atg gag act gat cag att gag gaa atg gat gtc gaa 1020
Met Val Cys Glu Met Glu Thr Asp Gln Ile Glu Glu Met Asp Val Glu
1               5                   10                  15
gtt ttg tct tcg atg tgg ccc gaa gat gtt gga act gaa gct gac aaa 1068
Val Leu Ser Ser Met Trp Pro Glu Asp Val Gly Thr Glu Ala Asp Lys
            20                  25                  30
cag ttc aac gtc gag aaa cct gcc gga gat tta gac acg ttg aaa gaa 1116
Gln Phe Asn Val Glu Lys Pro Ala Gly Asp Leu Asp Thr Leu Lys Glu
        35                  40                  45
gtt act atc gag aca cgg acc att gcg gat atg aca cgg tta cca aac 1164
Val Thr Ile Glu Thr Arg Thr Ile Ala Asp Met Thr Arg Leu Pro Asn
    50                  55                  60
cta ttg aat tcg act cat caa ggc tcc tct caa cta acc aac ctt gtg 1212
Leu Leu Asn Ser Thr His Gln Gly Ser Ser Gln Leu Thr Asn Leu Val
65                  70                  75                  80
aaa caa tgg gag tat atg caa gac aac gcg gtt cgg ctg tta aaa gaa 1260
Lys Gln Trp Glu Tyr Met Gln Asp Asn Ala Val Arg Leu Leu Lys Glu
                85                  90                  95
gag cta aaa aat ctc gat aga cag aga gaa gaa gcc gag gct aaa gag 1308
Glu Leu Lys Asn Leu Asp Arg Gln Arg Glu Glu Ala Glu Ala Lys Glu
            100                 105                 110
ttg aag atc att gag gag tat aag ttt gag agc aac gag cct gag aat 1356
Leu Lys Ile Ile Glu Glu Tyr Lys Phe Glu Ser Asn Glu Pro Glu Asn
        115                 120                 125
gtt ccg gtt ttg gat gag acg agt gat ttg ttc cgc agg ttt agg cag 1404
Val Pro Val Leu Asp Glu Thr Ser Asp Leu Phe Arg Arg Phe Arg Gln
    130                 135                 140
aaa aaa cga gat gcc ttg gtc gat agc aag aag att gag atc tat gag 1452
Lys Lys Arg Asp Ala Leu Val Asp Ser Lys Lys Ile Glu Ile Tyr Glu
145                 150                 155                 160
gag ttt gac act gtt gca tat tgg aaa cag aag gcg ttg agt ctt gag 1500
Glu Phe Asp Thr Val Ala Tyr Trp Lys Gln Lys Ala Leu Ser Leu Glu
                165                 170                 175
aaa atg ctt gag gcg agt act gag aga gaa agg cga ttg atg gag aag 1548
Lys Met Leu Glu Ala Ser Thr Glu Arg Glu Arg Arg Leu Met Glu Lys
            180                 185                 190

ctg agt gag agt ttg aaa act atg gag agt cag tca gca ccg gtc caa 1596
Leu Ser Glu Ser Leu Lys Thr Met Glu Ser Gln Ser Ala Pro Val Gln
        195                 200                 205
gag ctt act cag aat ctt aag aga gct gaa ggt ttc ttg cat ttc ata 1644
Glu Leu Thr Gln Asn Leu Lys Arg Ala Glu Gly Phe Leu His Phe Ile
    210                 215                 220
ctt cag aat gca cct att gtt atg ggc cat cag gtaaagtaaa aggtgattct 1697
Leu Gln Asn Ala Pro Ile Val Met Gly His Gln
225                 230                 235
tgatcttgtt ttcgatcttt tcgacttttc tgattctttg ttacttatga gtttcag 1754
gat aaa gat tta cgc tac ttg ttc atc tac aac aag tat cct agt tta 1802
Asp Lys Asp Leu Arg Tyr Leu Phe Ile Tyr Asn Lys Tyr Pro Ser Leu
                240                 245                 250
cgg gaa cag gttagaagaa aactttttct tctgttggat ttattgaatc 1851
Arg Glu Gln
attctctgag gaaatgtgtt taaatcaaaa tctgtttctt actatatttc ag gac 1906
Asp
255
att ttg gga aaa aca gac gtg gag ata ttc cat gga ggt gga gtt aaa 1954
Ile Leu Gly Lys Thr Asp Val Glu Ile Phe His Gly Gly Gly Val Lys
                260                 265                 270
gaa tct gaa gat ttc aag aga gaa gtt ctt gag aaa gga aaa gct tca 2002
Glu Ser Glu Asp Phe Lys Arg Glu Val Leu Glu Lys Gly Lys Ala Ser
            275                 280                 285
aag aga gag atc aca ttt act aca gat tta ttt gga tca aag acg ttt 2050
Lys Arg Glu Ile Thr Phe Thr Thr Asp Leu Phe Gly Ser Lys Thr Phe
        290                 295                 300
ttg ata tat gtt gag cct gtt tac aac aaa gct ggc gag aaa atc ggt 2098
Leu Ile Tyr Val Glu Pro Val Tyr Asn Lys Ala Gly Glu Lys Ile Gly
    305                 310                 315
ata aac tac atg gga atg gaa gta act gat cag gttagttagc taaagatttt 2151
Ile Asn Tyr Met Gly Met Glu Val Thr Asp Gln
320                 325                 330
tgaactattc tataatctat gttctcattt tcacatcttc actggatctt cctgtgttac 2211
ag gta gtg aaa agg gag aaa atg gcg aaa ctt aga gaa gat aac gct 2258
Val Val Lys Arg Glu Lys Met Ala Lys Leu Arg Glu Asp Asn Ala
                335                 340                 345

gtg aga aag gcg atg gaa tca gaa ctg aac aag act att cac att aca 2306
Val Arg Lys Ala Met Glu Ser Glu Leu Asn Lys Thr Ile His Ile Thr
                350                 355                 360
g gtttgttcaa gttaagcagt gaaagtttta gaaagattaa tgagaaaact 2357
agacttaggt gttgtgtttt ttcttttgca g ag gag aca atg aga gca aag cag 2411
Glu Glu Thr Met Arg Ala Lys Gln
            365
atg cta gcg acg atg tct cat gag ata agg tca cca ttg tca gga gta 2459
Met Leu Ala Thr Met Ser His Glu Ile Arg Ser Pro Leu Ser Gly Val
370                 375                 380                 385
gtg gga atg gct gag ata ctt tca act aca aaa ctg gat aaa gag caa 2507
Val Gly Met Ala Glu Ile Leu Ser Thr Thr Lys Leu Asp Lys Glu Gln
                390                 395                 400
aga cag ttg ttg aat gtc atg atc tct tct ggt gat ttg gtg ctt cag 2555
Arg Gln Leu Leu Asn Val Met Ile Ser Ser Gly Asp Leu Val Leu Gln
            405                 410                 415
cta atc aac gac att ctt gat ctc tcc aag gtt gaa tca g gtacaatata 2605
Leu Ile Asn Asp Ile Leu Asp Leu Ser Lys Val Glu Ser
        420                 425                 430
ctgttttcaa agtttttgat cttgtggtgt ggtcattgtt caaatcctcg attacatatg 2665
ttatttttg gtatttgtgt gttcttttta g gt gtg atg aga tta gaa gct aca 2719
Gly Val Met Arg Leu Glu Ala Thr
                435
aag ttt cga cca aga gaa gta gtg aag cat gtg cta cag aca gct gca 2767
Lys Phe Arg Pro Arg Glu Val Val Lys His Val Leu Gln Thr Ala Ala
    440                 445                 450
gca tcg ctg aag aaa tct ttg aca tta gaa gga aac att gca gat gat 2815
Ala Ser Leu Lys Lys Ser Leu Thr Leu Glu Gly Asn Ile Ala Asp Asp
455                 460                 465                 470
gtt cct att gag gttactacac atttcagaaa gagttaaatg tggcaaaagt 2867
Val Pro Ile Glu
ctttgtgatc ttaacttttt ttcctttaaa tgtttag gta gtt gga gat gtt cta 2922
Val Val Gly Asp Val Leu
475                 480
agg att agg cag atc ctc acc aat ttg ata ag gttctttacc tgattcctgt 2974
Arg Ile Arg Gln Ile Leu Thr Asn Leu Ile Ser
                485                 490

attatttgca taatgacaag tttcggttcc tgatttgttc aaacgttttg cag c aat 3031
Asn
gct atc aag ttt aca cat gaa gga aat gta gga atc aaa ctc caa gtg 3079
Ala Ile Lys Phe Thr His Glu Gly Asn Val Gly Ile Lys Leu Gln Val
        495                 500                 505
ata tca gaa cca tcc ttt gtg cgg gat aac gca ttg aac gca gac acc 3127
Ile Ser Glu Pro Ser Phe Val Arg Asp Asn Ala Leu Asn Ala Asp Thr
    510                 515                 520
gag gaa cac gaa caa aac ggt ttg acc gag act tca gtt tgg att tgc 3175
Glu Glu His Glu Gln Asn Gly Leu Thr Glu Thr Ser Val Trp Ile Cys
525                 530                 535                 540
tgt gac gta tgg gac act gga att gga atc cca g gcaagcaagc 3219
Cys Asp Val Trp Asp Thr Gly Ile Gly Ile Pro
                545                 550
aattctgaca aatgaacatg aaaagactaa aaaactccaa ctaacctata tattaacctg 3279
gttcatgttt tggtgttctt gtgcag aa aac gct ctt cca tgt ttg ttc aag 3331
Glu Asn Ala Leu Pro Cys Leu Phe Lys
            555                 560
aag tac atg caa gca agc gct gat cat gcc cgg aaa tac ggt ggg act 3379
Lys Tyr Met Gln Ala Ser Ala Asp His Ala Arg Lys Tyr Gly Gly Thr
                565                 570                 575
ggt ctc gga ctt gct att tgt aaa cag ctg gtaagctatt attaggattt ta 3431
Gly Leu Gly Leu Ala Ile Cys Lys Gln Leu
            580                 585
caacacta cagaagaaac taatgaagct cgttatccta atctttcttt gtttgtttac 3489
ag gtt gag tta atg gga ggc caa ctc act gtg aca agc cgg gtg agc 3536
Val Glu Leu Met Gly Gly Gln Leu Thr Val Thr Ser Arg Val Ser
            590                 595                 600
gaa ggt tca acg ttc aca ttt ata tta ccc tac aaa gtt gga aga tca 3586
Glu Gly Ser Thr Phe Thr Phe Ile Leu Pro Tyr Lys Val Gly Arg Ser
            605                 610                 615
gat gat tat tca gat gat caa gat gag ttc tct gat atg gcg gat caa 3632
Asp Asp Tyr Ser Asp Asp Gln Asp Glu Phe Ser Asp Met Ala Asp Gln
        620                 625                 630
caa tct gaa cca gac gat aca gct gaa gga tat ttc cag ttt aaa ccg 3680
Gln Ser Glu Pro Asp Asp Thr Ala Glu Gly Tyr Phe Gln Phe Lys Pro
    635                 640                 645

ctc tta gga tcg ata tat tcg aat ggc gga ccg ggg atc agc aat gac 3728
Leu Leu Gly Ser Ile Tyr Ser Asn Gly Gly Pro Gly Ile Ser Asn Asp
650                 655                 660                 665
ttc tta cct cat aaa gtc atg ctt act agt cct att aag ctc atc aat 3776
Phe Leu Pro His Lys Val Met Leu Thr Ser Pro Ile Lys Leu Ile Asn
                670                 675                 680
ggt ttt gtc gct gat ccc tct aat aac act gga cag agc gag atg cta 3824
Gly Phe Val Ala Asp Pro Ser Asn Asn Thr Gly Gln Ser Glu Met Leu
            685                 690                 695
cag ctt gaa aac ggt ggt tac atg gat gaa tct aaa ctc gaa acc agt 3872
Gln Leu Glu Asn Gly Gly Tyr Met Asp Glu Ser Lys Leu Glu Thr Ser
        700                 705                 710
tct ggt cat tgc cct gaa tca gct cac caa tat gag aat gga aat ggt 3920
Ser Gly His Cys Pro Glu Ser Ala His Gln Tyr Glu Asn Gly Asn Gly
    715                 720                 725
cga tgt ttc tct aag gaa tct gaa tct tgt agc agt tca caa gct agc 3968
Arg Cys Phe Ser Lys Glu Ser Glu Ser Cys Ser Ser Ser Gln Ala Ser
730                 735                 740                 745
tca gaa ggt gga acc tta gaa atg gag tca gag ctc aca gtt tca tct 4016
Ser Glu Gly Gly Thr Leu Glu Met Glu Ser Glu Leu Thr Val Ser Ser
                750                 755                 760
cat agg gaa gag gaa aaa gcc gag aca gaa gta aaa gaa aca tca aag 4064
His Arg Glu Glu Glu Lys Ala Glu Thr Glu Val Lys Glu Thr Ser Lys
            765                 770                 775
cca aag att ttg ctt gtg gaa gat aat aag atc aac atc atg gtt gca 4112
Pro Lys Ile Leu Leu Val Glu Asp Asn Lys Ile Asn Ile Met Val Ala
        780                 785                 790
aag tcg atg atg aag cag tta ggc cat acc atg gat att gct aat aat 4160
Lys Ser Met Met Lys Gln Leu Gly His Thr Met Asp Ile Ala Asn Asn
    795                 800                 805
gga gtt gaa gcc ata acc gcg att aat agc tct agc tac gat ctg gta 4208
Gly Val Glu Ala Ile Thr Ala Ile Asn Ser Ser Ser Tyr Asp Leu Val
810                 815                 820                 825
ctc atg gtatgtaaat tttcttagct ctaaggactt gtccttttca aattcactta 4264
Leu Met
tatttgaaaa aggcttataa ttcatattgg gttcacag gat gtg tgc atg ccg gtg 4320
Asp Val Cys Met Pro Val
        830

ctc gat ggt tta aaa gct aca aga ctg atc cgt tcg tat gaa gaa act    4368
Leu Asp Gly Leu Lys Ala Thr Arg Leu Ile Arg Ser Tyr Glu Glu Thr
    835                 840                 845
ggg aac tgg aat gct gca ata gaa gcc gga gta gat ata tcg aca tcg    4416
Gly Asn Trp Asn Ala Ala Ile Glu Ala Gly Val Asp Ile Ser Thr Ser
850                 855                 860                 865
gag aat gaa caa gtt tgt atg cgt ccc aca aac cgg ctg cct ata atc    4464
Glu Asn Glu Gln Val Cys Met Arg Pro Thr Asn Arg Leu Pro Ile Ile
                870                 875                 880
gcg gtcagtactt cttttttgaa tactaaacac agagatctaa tgcataactt          4517
Ala
gagaaaactg atagtcagaa tgcgttgtgg aatgtgcag atg acg gca aat act    4571
                                           Met Thr Ala Asn Thr
                                                   885
tta gca gag agt tca gaa gaa tgt tat gca aat ggt atg gac tcg ttt    4619
Leu Ala Glu Ser Ser Glu Glu Cys Tyr Ala Asn Gly Met Asp Ser Phe
        890                 895                 900
att tcg aaa cct gta acg ttg caa aaa ctg aga gag tgt ttg caa cag    4667
Ile Ser Lys Pro Val Thr Leu Gln Lys Leu Arg Glu Cys Leu Gln Gln
    905                 910                 915
tat ttg cac tga                                                    4679
Tyr Leu His
920
<210>    4
<211>    922
<212>    PRT
<213>    Arabidopsis thariana
<223>    Amino acid sequence of histidine protein kinese
<400>    4
Met Val Cys Glu Met Glu Thr Asp Gln Ile Glu Glu Met Asp Val Glu
  1               5                  10                  15
Val Leu Ser Ser Met Trp Pro Glu Asp Val Gly Thr Glu Ala Asp Lys
             20                  25                  30
Gln Phe Asn Val Glu Lys Pro Ala Gly Asp Leu Asp Thr Leu Lys Glu
         35                  40                  45
Val Thr Ile Glu Thr Arg Thr Ile Ala Asp Met Thr Arg Leu Pro Asn
     50                  55                  60

Leu Leu Asn Ser Thr His Gln Gly Ser Ser Gln Leu Thr Asn Leu Val
65                  70                  75                  80
Lys Gln Trp Glu Tyr Met Gln Asp Asn Ala Val Arg Leu Leu Lys Glu
                85                  90                  95
Glu Leu Lys Asn Leu Asp Arg Gln Arg Glu Glu Ala Glu Ala Lys Glu
            100                 105                 110
Leu Lys Ile Ile Glu Glu Tyr Lys Phe Glu Ser Asn Glu Pro Glu Asn
        115                 120                 125
Val Pro Val Leu Asp Glu Thr Ser Asp Leu Phe Arg Arg Phe Arg Gln
    130                 135                 140
Lys Lys Arg Asp Ala Leu Val Asp Ser Lys Lys Ile Glu Ile Tyr Glu
145                 150                 155                 160
Glu Phe Asp Thr Val Ala Tyr Trp Lys Gln Lys Ala Leu Ser Leu Glu
                165                 170                 175
Lys Met Leu Glu Ala Ser Thr Glu Arg Glu Arg Arg Leu Met Glu Lys
            180                 185                 190
Leu Ser Glu Ser Leu Lys Thr Met Glu Ser Gln Ser Ala Pro Val Gln
        195                 200                 205
Glu Leu Thr Gln Asn Leu Lys Arg Ala Glu Gly Phe Leu His Phe Ile
    210                 215                 220
Leu Gln Asn Ala Pro Ile Val Met Gly His Gln Asp Lys Asp Leu Arg
225                 230                 235                 240
Tyr Leu Phe Ile Tyr Asn Lys Tyr Pro Ser Leu Arg Glu Gln Asp Ile
                245                 250                 255
Leu Gly Lys Thr Asp Val Glu Ile Phe His Gly Gly Gly Val Lys Glu
            260                 265                 270
Ser Glu Asp Phe Lys Arg Glu Val Leu Glu Lys Gly Lys Ala Ser Lys
        275                 280                 285
Arg Glu Ile Thr Phe Thr Thr Asp Leu Phe Gly Ser Lys Thr Phe Leu
    290                 295                 300
Ile Tyr Val Glu Pro Val Tyr Asn Lys Ala Gly Glu Lys Ile Gly Ile
305                 310                 315                 320
Asn Tyr Met Gly Met Glu Val Thr Asp Gln Val Val Lys Arg Glu Lys
                325                 330                 335
Met Ala Lys Leu Arg Glu Asp Asn Ala Val Arg Lys Ala Met Glu Ser
            340                 345                 350
Glu Leu Asn Lys Thr Ile His Ile Thr Glu Glu Thr Met Arg Ala Lys
        355                 360                 365

Gln Met Leu Ala Thr Met Ser His Glu Ile Arg Ser Pro Leu Ser Gly
    370                 375                 380
Val Val Gly Met Ala Glu Ile Leu Ser Thr Thr Lys Leu Asp Lys Glu
385                 390                 395                 400
Gln Arg Gln Leu Leu Asn Val Met Ile Ser Ser Gly Asp Leu Val Leu
                405                 410                 415
Gln Leu Ile Asn Asp Ile Leu Asp Leu Ser Lys Val Glu Ser Gly Val
            420                 425                 430
Met Arg Leu Glu Ala Thr Lys Phe Arg Pro Arg Glu Val Val Lys His
        435                 440                 445
Val Leu Gln Thr Ala Ala Ala Ser Leu Lys Lys Ser Leu Thr Leu Glu
    450                 455                 460
Gly Asn Ile Ala Asp Asp Val Pro Ile Glu Val Val Gly Asp Val Leu
465                 470                 475                 480
Arg Ile Arg Gln Ile Leu Thr Asn Leu Ile Ser Asn Ala Ile Lys Phe
                485                 490                 495
Thr His Glu Gly Asn Val Gly Ile Lys Leu Gln Val Ile Ser Glu Pro
            500                 505                 510
Ser Phe Val Arg Asp Asn Ala Leu Asn Ala Asp Thr Glu Glu His Glu
        515                 520                 525
Gln Asn Gly Leu Thr Glu Thr Ser Val Trp Ile Cys Cys Asp Val Trp
    530                 535                 540
Asp Thr Gly Ile Gly Ile Pro Glu Asn Ala Leu Pro Cys Leu Phe Lys
545                 550                 555                 560
Lys Tyr Met Gln Ala Ser Ala Asp His Ala Arg Lys Tyr Gly Gly Thr
                565                 570                 575
Gly Leu Gly Leu Ala Ile Cys Lys Gln Leu Val Glu Leu Met Gly Gly
            580                 585                 590
Gln Leu Thr Val Thr Ser Arg Val Ser Glu Gly Ser Thr Phe Thr Phe
        595                 600                 605
Ile Leu Pro Tyr Lys Val Gly Arg Ser Asp Asp Tyr Ser Asp Asp Gln
    610                 615                 620
Asp Glu Phe Ser Asp Met Ala Asp Gln Gln Ser Glu Pro Asp Asp Thr
625                 630                 635                 640
Ala Glu Gly Tyr Phe Gln Phe Lys Pro Leu Leu Gly Ser Ile Tyr Ser
                645                 650                 655
Asn Gly Gly Pro Gly Ile Ser Asn Asp Phe Leu Pro His Lys Val Met
            660                 665                 670

Leu Thr Ser Pro Ile Lys Leu Ile Asn Gly Phe Val Ala Asp Pro Ser
        675                 680                 685
Asn Asn Thr Gly Gln Ser Glu Met Leu Gln Leu Glu Asn Gly Gly Tyr
    690                 695                 700
Met Asp Glu Ser Lys Leu Glu Thr Ser Ser Gly His Cys Pro Glu Ser
705                 710                 715                 720
Ala His Gln Tyr Glu Asn Gly Asn Gly Arg Cys Phe Ser Lys Glu Ser
                725                 730                 735
Glu Ser Cys Ser Ser Ser Gln Ala Ser Ser Glu Gly Gly Thr Leu Glu
            740                 745                 750
Met Glu Ser Glu Leu Thr Val Ser Ser His Arg Glu Glu Glu Lys Ala
        755                 760                 765
Glu Thr Glu Val Lys Glu Thr Ser Lys Pro Lys Ile Leu Leu Val Glu
    770                 775                 780
Asp Asn Lys Ile Asn Ile Met Val Ala Lys Ser Met Met Lys Gln Leu
785                 790                 795                 800
Gly His Thr Met Asp Ile Ala Asn Asn Gly Val Glu Ala Ile Thr Ala
                805                 810                 815
Ile Asn Ser Ser Ser Tyr Asp Leu Val Leu Met Asp Val Cys Met Pro
            820                 825                 830
Val Leu Asp Gly Leu Lys Ala Thr Arg Leu Ile Arg Ser Tyr Glu Glu
        835                 840                 845
Thr Gly Asn Trp Asn Ala Ala Ile Glu Ala Gly Val Asp Ile Ser Thr
    850                 855                 860
Ser Glu Asn Glu Gln Val Cys Met Arg Pro Thr Asn Arg Leu Pro Ile
865                 870                 875                 880
Ile Ala Met Thr Ala Asn Thr Leu Ala Glu Ser Ser Glu Glu Cys Tyr
                885                 890                 895
Ala Asn Gly Met Asp Ser Phe Ile Ser Lys Pro Val Thr Leu Gln Lys
            900                 905                 910
Leu Arg Glu Cys Leu Gln Gln Tyr Leu His
        915                 920
<210> 5
<211> 3708
<212> DNA
<213> Arabidopsis thaliana
<220>
<221> exon 1

<222>      (1) ··· (705)
<221>      exon 2
<222>      (783) ··· (839)
<221>      exon 3
<222>      (933) ··· (1160)
<221>      exon 4
<222>      (1243) ··· (1336)
<221>      exon 5
<222>      (1418) ··· (1624)
<221>      exon 6
<222>      (1726) ··· (1856)
<221>      exon 7
<222>      (1934) ··· (1983)
<221>      exon 8
<222>      (2057) ··· (2238)
<221>      exon 9
<222>      (2335) ··· (2438)
<221>      exon 10
<222>      (2521) ··· (3243)
<221>      exon 11
<222>      (3332) ··· (3496)
<221>      exon 12
<222>      (3586) ··· (3708)
<223>      Nucleotide sequence of genomic DNA coding for hist
idine protein kinase
<400>      5
   atg gtc tgt gaa atg gag act gat cag att gag gaa atg gat gtc gaa      48
   Met Val Cys Glu Met Glu Thr Asp Gln Ile Glu Glu Met Asp Val Glu
    1               5                  10                  15
   gtt ttg tct tcg atg tgg ccc gaa gat gtt gga act gaa gct gac aaa      96
   Val Leu Ser Ser Met Trp Pro Glu Asp Val Gly Thr Glu Ala Asp Lys
               20                  25                  30
   cag ttc aac gtc gag aaa cct gcc gga gat tta gac acg ttg aaa gaa      144
   Gln Phe Asn Val Glu Lys Pro Ala Gly Asp Leu Asp Thr Leu Lys Glu
           35                  40                  45
   gtt act atc gag aca cgg acc att gcg gat atg aca cgg tta cca aac 192
   Val Thr Ile Glu Thr Arg Thr Ile Ala Asp Met Thr Arg Leu Pro Asn
   50 55 60
   cta ttg aat tcg act cat caa ggc tcc tct caa cta acc aac ctt gtg 240
   Leu Leu Asn Ser Thr His Gln Gly Ser Ser Gln Leu Thr Asn Leu Val
   65 70 75 80
   aaa caa tgg gag tat atg caa gac aac gcg gtt cgg ctg tta aaa gaa 288
   Lys Gln Trp Glu Tyr Met Gln Asp Asn Ala Val Arg Leu Leu Lys Glu
   85 90 95
   gag cta aag aat ctc gat aga cag aga gaa gaa gct gag gct aaa gag 336
   Glu Leu Lys Asn Leu Asp Arg Gln Arg Glu Glu Ala Glu Ala Lys Glu
   100 105 110
   ttg aag atc att gag gag tat aag ttt gag agc aac gag cct gag aat 384
   Leu Lys Ile Ile Glu Glu Tyr Lys Phe Glu Ser Asn Glu Pro Glu Asn
   115 120 125
   gtt ccg gtt ttg gat gag acg agt gat ttg ttc cgc agg ttt agg cag 432
   Val Pro Val Leu Asp Glu Thr Ser Asp Leu Phe Arg Arg Phe Arg Gln
   130 135 140
   aaa aaa cga gat gcc ttg gtc gat agc aag aag att gag atc tat gag 480
   Lys Lys Arg Asp Ala Leu Val Asp Ser Lys Lys Ile Glu Ile Tyr Glu
   145 150 155 160
   gag ttt gac act gtt gca tat tgg aaa cag aag gcg ttg agt ctt gag 528
   Glu Phe Asp Thr Val Ala Tyr Trp Lys Gln Lys Ala Leu Ser Leu Glu
   165 170 175
   aaa atg ctt gag gcg agt act gag aga gaa agg cga ttg atg gag aag 576
   Lys Met Leu Glu Ala Ser Thr Glu Arg Glu Arg Arg Leu Met Glu Lys
   180 185 190
   ctg agt gag agt ttg aaa act atg gag agt cag tca gca ccg gtc caa 624
   Leu Ser Glu Ser Leu Lys Thr Met Glu Ser Gln Ser Ala Pro Val Gln
   195 200 205
   gag ctt act cag aat ctt aag aga gct gaa ggt ttc ttg cat ttc ata 672
   Glu Leu Thr Gln Asn Leu Lys Arg Ala Glu Gly Phe Leu His Phe Ile
   210 215 220
   ctt cag aat gca cct att gtt atg ggc cat cag gtaaagtaaa aggtgattct 725
   Leu Gln Asn Ala Pro Ile Val Met Gly His Gln
   225 230 235
   tgatcttgtt ttcgatcttt tcgacttttc tgattctttg ttacttatga gtttcag 782
   gat aaa gat tta cgc tac ttg ttc atc tac aac aag tat cct agt tta 830
   Asp Lys Asp Leu Arg Tyr Leu Phe Ile Tyr Asn Lys Tyr Pro Ser Leu
   240 245 250
   cgg gaa cag gttagaagaa aactttttct tctgttggat ttattgaatc 879
   Arg Glu Gln
   attctcttga ggaaatgtgt ttaaatcaaa atctgtttct tactatattt cag gac 935
   Asp
   255
   att ttg gga aaa aca gac gtg gag ata ttc cat gga ggt gga gtt aaa 983
   Ile Leu Gly Lys Thr Asp Val Glu Ile Phe His Gly Gly Gly Val Lys
   260 265 270
   gaa tct gaa gat ttc aag aga gaa gtt ctt gag aaa gga aaa gct tca 1031
   Glu Ser Glu Asp Phe Lys Arg Glu Val Leu Glu Lys Gly Lys Ala Ser
   275 280 285
   aag aga gaa atc aca ttt act aca gat tta ttt gga tca aag acg ttt 1079
   Lys Arg Glu Ile Thr Phe Thr Thr Asp Leu Phe Gly Ser Lys Thr Phe
   290 295 300
   ttg ata tat gtt gag cct gtt tac aac aaa gct ggc gag aaa atc ggt 1127
   Leu Ile Tyr Val Glu Pro Val Tyr Asn Lys Ala Gly Glu Lys Ile Gly
   305 310 315
   ata aac tac atg gga atg gaa gta act gat cag gttagttagc taaagatttt 1180
   Ile Asn Tyr Met Gly Met Glu Val Thr Asp Gln
   320 325 330
   tgaactattc tataatctat gttctcattt tcacatcttc actggatctt cctgtgttac 1240
   ag gta gtg aaa agg gag aaa atg gcg aaa ctt aga gaa gat aac gct 1287
   Val Val Lys Arg Glu Lys Met Ala Lys Leu Arg Glu Asp Asn Ala
   335 340 345
   gtg aga aag gcg atg gaa tca gaa ctg aac aag act att cac att aca 1335
   Val Arg Lys Ala Met Glu Ser Glu Leu Asn Lys Thr Ile His Ile Thr
   350 355 360
   g gtttgttcaa gttaagcact gaaagtttta gaaagattaa tgagaaaact 1386
   agacttaggt gttgtgtttt ttcttttgca g ag gag aca atg aga gca aag cag 1440
   Glu Glu Thr Met Arg Ala Lys Gln
   365
   atg cta gcg acg atg tct cat gag ata agg tca cca ttg tca gga gta 1488
   Met Leu Ala Thr Met Ser His Glu Ile Arg Ser Pro Leu Ser Gly Val
   370 375 380 385
   gtg gga atg gct gag ata ctt tca act aca aaa ctg gat aaa gag caa 1536
   Val Gly Met Ala Glu Ile Leu Ser Thr Thr Lys Leu Asp Lys Glu Gln
   390 395 400
   aga cag ttg ttg aat gtc atg atc tct tct ggt gat ttg gtg ctt cag 1584
   Arg Gln Leu Leu Asn Val Met Ile Ser Ser Gly Asp Leu Val Leu Gln
   405 410 415
   cta atc aac gac att ctt gat ctc tcc aag gtt gaa tca g gtacaatata 1634
   Leu Ile Asn Asp Ile Leu Asp Leu Ser Lys Val Glu Ser
   420 425 430
   ctgttttcaa agtttttgat cttgtggtgt ggtcattgtt caaatcctcg attacatatg 1694
   ttattttttg gtatttgtgt gttcttttta g gt gtg atg aga tta gaa gct aca 1748
   Gly Val Met Arg Leu Glu Ala Thr
   435
   aag ttt cga cca aga gaa gta gtg aag cat gtg cta cag aca gct gca 1796
   Lys Phe Arg Pro Arg Glu Val Val Lys His Val Leu Gln Thr Ala Ala
   440 445 450
   gca tcg ctg aag aaa tct ttg aca tta gaa gga aac att gca gat gat 1844
   Ala Ser Leu Lys Lys Ser Leu Thr Leu Glu Gly Asn Ile Ala Asp Asp
   455 460 465 470
   gtt cct att gag gttactacac atttcagaaa gagttaaatg tggcaaaagt 1896
   Val Pro Ile Glu
   ctttgtgatc ttaacttttt ttcctttaaa tgtttag gta gtt gga gat gtt 1948
   Val Val Gly Asp Val
   475
   cta agg att agg cag atc ctc acc aat ttg ata ag gttctttacc 1993
   Leu Arg Ile Arg Gln Ile Leu Thr Asn Leu Ile Ser
   480 485 490
   tgattcctgt attatttgca taatgacaag ttttggttcc tgatttgttc aaacgttttg 2053
   cag c aat gct atc aag ttt aca cat gaa gga aat gta gga atc aaa 2099
   Asn Ala Ile Lys Phe Thr His Glu Gly Asn Val Gly Ile Lys
   495 500 505
   ctc caa gtg ata tca gaa cca tcc ttt gtg cgg gat aac gca ttg aac 2147
   Leu Gln Val Ile Ser Glu Pro Ser Phe Val Arg Asp Asn Ala Leu Asn
   510 515 520
   gca gac acc gag gaa cac gaa caa aac ggt ttg acc gag act tca gtt 2195
   Ala Asp Thr Glu Glu His Glu Gln Asn Gly Leu Thr Glu Thr Ser Val
   525 530 535
   tgg att tgc tgt gac gta tgg gac act gga att gga atc cca 2237
   Trp Ile Cys Cys Asp Val Trp Asp Thr Gly Ile Gly Ile Pro
   540 545 550
   g gcaagcaagc aattctgaca aatgaacatg aaaagactaa aaaactccaa 2288
   ctaacctata tattaacctg gttcatgttt tggtgttctt gtgcag aa aac 2339
   Glu Asn
   gct ctt cca tgt ttg ttc aag aag tac atg caa gca agc gct gat cat 2387
   Ala Leu Pro Cys Leu Phe Lys Lys Tyr Met Gln Ala Ser Ala Asp His
   555 560 565
   gcc cga aaa tac ggt ggg act ggt ctc gga ctt gct att tgt aaa cag 2435
   Ala Arg Lys Tyr Gly Gly Thr Gly Leu Gly Leu Ala Ile Cys Lys Gln
   570 575 580 585
   ctg gtaagctatt attaggattt tacaacacta cagaagaaac taatgaagct 2488
   Leu
   cgttatccta atctttctct gtttgtttac ag gtt gag tta atg gga ggc caa 2541
   Val Glu Leu Met Gly Gly Gln
   590
   ctc act gtg aca agc cgg gtg aac gaa ggt tca acg ttc aca ttt ata 2589
   Leu Thr Val Thr Ser Arg Val Asn Glu Gly Ser Thr Phe Thr Phe Ile
   595 600 605
   tta ccc tac aaa gtt gga aga tca gat gat tat tca gat gat caa gat 2637
   Leu Pro Tyr Lys Val Gly Arg Ser Asp Asp Tyr Ser Asp Asp Gln Asp
   610 615 620 625
   gag ttc tct gat atg gcg gat caa caa tct gaa cca gac gat aca gct 2685
   Glu Phe Ser Asp Met Ala Asp Gln Gln Ser Glu Pro Asp Asp Thr Ala
   630 635 640
   gaa gga tat ttc cag ttt aaa ccg ctc tta gga tcg ata tat tcg aat 2733
   Glu Gly Tyr Phe Gln Phe Lys Pro Leu Leu Gly Ser Ile Tyr Ser Asn
   645 650 655
   ggc gga ccg ggg atc agc aat gac ttc tta cct cat aaa gtc atg ctt 2781
   Gly Gly Pro Gly Ile Ser Asn Asp Phe Leu Pro His Lys Val Met Leu
   660 665 670
   act agt cct att aag ctc atc aat ggt ttt gtc gct gat ccc tct aac 2829
   Thr Ser Pro Ile Lys Leu Ile Asn Gly Phe Val Ala Asp Pro Ser Asn
   675 680 685
   aac act gga cag agc gag atg cta cag ctt gaa aac ggt ggt tac atg 2877
   Asn Thr Gly Gln Ser Glu Met Leu Gln Leu Glu Asn Gly Gly Tyr Met
   690 695 700 705
   gat gaa tct aaa ctc gaa acc agt tct ggt cat tgc cct gaa tca gct 2925
   Asp Glu Ser Lys Leu Glu Thr Ser Ser Gly His Cys Pro Glu Ser Ala
   710 715 720
   cac caa tat gag aat gga aat ggt cga tgt ttc tct aag gaa tct gaa 2973
   His Gln Tyr Glu Asn Gly Asn Gly Arg Cys Phe Ser Lys Glu Ser Glu
   725 730 735
   tct tgt agc agt tca caa gct agc tca gaa ggt gga acc tta gaa atg 3021
   Ser Cys Ser Ser Ser Gln Ala Ser Ser Glu Gly Gly Thr Leu Glu Met
   740 745 750
   gag tca gag ctc aca gtt tca tct cat agg gaa gag gaa aaa gcc gag 3069
   Glu Ser Glu Leu Thr Val Ser Ser His Arg Glu Glu Glu Lys Ala Glu
   755 760 765
   ata gaa gta aaa gaa aca tca aag cca aat att ttg ctt gtg gaa gat 3117
   Ile Glu Val Lys Glu Thr Ser Lys Pro Asn Ile Leu Leu Val Glu Asp
   770 775 780 785
   aat aag atc aac atc atg gtt gca aag tcg atg atg aag cag tta ggc 3165
   Asn Lys Ile Asn Ile Met Val Ala Lys Ser Met Met Lys Gln Leu Gly
   790 795 800
   cat acc atg gat att gct aat aat gga gtt gaa gcc ata acc gcg att 3213
   His Thr Met Asp Ile Ala Asn Asn Gly Val Glu Ala Ile Thr Ala Ile
   805 810 815
   aat agc tct agc tac gat ctg gta ctc atg gtatgtaaat tttcttagct 3263
   Asn Ser Ser Ser Tyr Asp Leu Val Leu Met
   820 825
   ctaaggactt gtccttttca aattcactta tatttgaaaa aggcttataa ttcatattgg 3323
   gttcacag gat gtg tgc atg ccg gtg ctc gat ggt tta aaa gct aca 3370
   Asp Val Cys Met Pro Val Leu Asp Gly Leu Lys Ala Thr
   830 835 840
   aga ctg atc cgt tcg tat gaa gaa act ggg aac tgg aat gct gca ata 3418
   Arg Leu Ile Arg Ser Tyr Glu Glu Thr Gly Asn Trp Asn Ala Ala Ile
   845 850 855
   gaa gcc gga gta gat ata tcg aca tcg gag aat gaa caa gtt tgt atg 3466
   Glu Ala Gly Val Asp Ile Ser Thr Ser Glu Asn Glu Gln Val Cys Met
   860 865 870
   cgt ccc aca aac cgg ctg cct ata atc gcg gtcagtactt ctttttcgaa 3516
   Arg Pro Thr Asn Arg Leu Pro Ile Ile Ala
   875 880
   tactaaacac agagatctaa tgcataactt gagaaaactg atagtcagaa tgcgttgtgg 3576
   aatgtgcag atg acg gca aat act tta gca gag agt tca gaa gaa tgt 3624
   Met Thr Ala Asn Thr Leu Ala Glu Ser Ser Glu Glu Cys
   885 890 895
   tat gca aat ggt atg gac tcg ttt att tcg aaa cct gta acg ttg caa 3672
   Tyr Ala Asn Gly Met Asp Ser Phe Ile Ser Lys Pro Val Thr Leu Gln
   900 905 910
   aaa ctg aga gag tgt ttg caa cag tat ttg cac tga 3708
   Lys Leu Arg Glu Cys Leu Gln Gln Tyr Leu His
   915 920
<210> 6
<211> 922
<212> PRT
<213> Arabidopsis thariana
<223> Amino acid sequence of histidine protein kinese
<400> 6
Met Val Cys Glu Met Glu Thr Asp Gln Ile Glu Glu Met Asp Val Glu
1               5                   10                  15
Val Leu Ser Ser Met Trp Pro Glu Asp Val Gly Thr Glu Ala Asp Lys
            20                  25                  30
Gln Phe Asn Val Glu Lys Pro Ala Gly Asp Leu Asp Thr Leu Lys Glu
        35                  40                  45
Val Thr Ile Glu Thr Arg Thr Ile Ala Asp Met Thr Arg Leu Pro Asn
    50                  55                  60
Leu Leu Asn Ser Thr His Gln Gly Ser Ser Gln Leu Thr Asn Leu Val
65                  70                  75                  80
Lys Gln Trp Glu Tyr Met Gln Asp Asn Ala Val Arg Leu Leu Lys Glu
                85                  90                  95
Glu Leu Lys Asn Leu Asp Arg Gln Arg Glu Glu Ala Glu Ala Lys Glu
            100                 105                 110
Leu Lys Ile Ile Glu Glu Tyr Lys Phe Glu Ser Asn Glu Pro Glu Asn
        115                 120                 125
Val Pro Val Leu Asp Glu Thr Ser Asp Leu Phe Arg Arg Phe Arg Gln
    130                 135                 140
Lys Lys Arg Asp Ala Leu Val Asp Ser Lys Lys Ile Glu Ile Tyr Glu
145                 150                 155                 160
Glu Phe Asp Thr Val Ala Tyr Trp Lys Gln Lys Ala Leu Ser Leu Glu
                165                 170                 175

Lys Met Leu Glu Ala Ser Thr Glu Arg Glu Arg Arg Leu Met Glu Lys
            180                 185                 190
Leu Ser Glu Ser Leu Lys Thr Met Glu Ser Gln Ser Ala Pro Val Gln
        195                 200                 205
Glu Leu Thr Gln Asn Leu Lys Arg Ala Glu Gly Phe Leu His Phe Ile
    210                 215                 220
Leu Gln Asn Ala Pro Ile Val Met Gly His Gln Asp Lys Asp Leu Arg
225                 230                 235                 240
Tyr Leu Phe Ile Tyr Asn Lys Tyr Pro Ser Leu Arg Glu Gln Asp Ile
                245                 250                 255
Leu Gly Lys Thr Asp Val Glu Ile Phe His Gly Gly Gly Val Lys Glu
            260                 265                 270
Ser Glu Asp Phe Lys Arg Glu Val Leu Glu Lys Gly Lys Ala Ser Lys
        275                 280                 285
Arg Glu Ile Thr Phe Thr Thr Asp Leu Phe Gly Ser Lys Thr Phe Leu
    290                 295                 300
Ile Tyr Val Glu Pro Val Tyr Asn Lys Ala Gly Glu Lys Ile Gly Ile
305                 310                 315                 320
Asn Tyr Met Gly Met Glu Val Thr Asp Gln Val Val Lys Arg Glu Lys
                325                 330                 335
Met Ala Lys Leu Arg Glu Asp Asn Ala Val Arg Lys Ala Met Glu Ser
            340                 345                 350
Glu Leu Asn Lys Thr Ile His Ile Thr Glu Glu Thr Met Arg Ala Lys
        355                 360                 365
Gln Met Leu Ala Thr Met Ser His Glu Ile Arg Ser Pro Leu Ser Gly
    370                 375                 380
Val Val Gly Met Ala Glu Ile Leu Ser Thr Thr Lys Leu Asp Lys Glu
385                 390                 395                 400
Gln Arg Gln Leu Leu Asn Val Met Ile Ser Ser Gly Asp Leu Val Leu
                405                 410                 415
Gln Leu Ile Asn Asp Ile Leu Asp Leu Ser Lys Val Glu Ser Gly Val
            420                 425                 430
Met Arg Leu Glu Ala Thr Lys Phe Arg Pro Arg Glu Val Val Lys His
        435                 440                 445
Val Leu Gln Thr Ala Ala Ala Ser Leu Lys Lys Ser Leu Thr Leu Glu
    450                 455                 460
Gly Asn Ile Ala Asp Asp Val Pro Ile Glu Val Val Gly Asp Val Leu
465                 470                 475                 480

Arg Ile Arg Gln Ile Leu Thr Asn Leu Ile Ser Asn Ala Ile Lys Phe
                485                 490                 495
Thr His Glu Gly Asn Val Gly Ile Lys Leu Gln Val Ile Ser Glu Pro
            500                 505                 510
Ser Phe Val Arg Asp Asn Ala Leu Asn Ala Asp Thr Glu Glu His Glu
        515                 520                 525
Gln Asn Gly Leu Thr Glu Thr Ser Val Trp Ile Cys Cys Asp Val Trp
    530                 535                 540
Asp Thr Gly Ile Gly Ile Pro Glu Asn Ala Leu Pro Cys Leu Phe Lys
545                 550                 555                 560
Lys Tyr Met Gln Ala Ser Ala Asp His Ala Arg Lys Tyr Gly Gly Thr
                565                 570                 575
Gly Leu Gly Leu Ala Ile Cys Lys Gln Leu Val Glu Leu Met Gly Gly
            580                 585                 590
Gln Leu Thr Val Thr Ser Arg Val Asn Glu Gly Ser Thr Phe Thr Phe
        595                 600                 605
Ile Leu Pro Tyr Lys Val Gly Arg Ser Asp Asp Tyr Ser Asp Asp Gln
    610                 615                 620
Asp Glu Phe Ser Asp Met Ala Asp Gln Gln Ser Glu Pro Asp Asp Thr
625                 630                 635                 640
Ala Glu Gly Tyr Phe Gln Phe Lys Pro Leu Leu Gly Ser Ile Tyr Ser
                645                 650                 655
Asn Gly Gly Pro Gly Ile Ser Asn Asp Phe Leu Pro His Lys Val Met
            660                 665                 670
Leu Thr Ser Pro Ile Lys Leu Ile Asn Gly Phe Val Ala Asp Pro Ser
        675                 680                 685
Asn Asn Thr Gly Gln Ser Glu Met Leu Gln Leu Glu Asn Gly Gly Tyr
    690                 695                 700
Met Asp Glu Ser Lys Leu Glu Thr Ser Ser Gly His Cys Pro Glu Ser
705                 710                 715                 720
Ala His Gln Tyr Glu Asn Gly Asn Gly Arg Cys Phe Ser Lys Glu Ser
                725                 730                 735
Glu Ser Cys Ser Ser Ser Gln Ala Ser Ser Glu Gly Gly Thr Leu Glu
            740                 745                 750
Met Glu Ser Glu Leu Thr Val Ser Ser His Arg Glu Glu Glu Lys Ala
        755                 760                 765
Glu Ile Glu Val Lys Glu Thr Ser Lys Pro Asn Ile Leu Leu Val Glu
    770                 775                 780

Asp Asn Lys Ile Asn Ile Met Val Ala Lys Ser Met Met Lys Gln Leu
785                 790                 795                 800
Gly His Thr Met Asp Ile Ala Asn Asn Gly Val Glu Ala Ile Thr Ala
                805                 810                 815
Ile Asn Ser Ser Ser Tyr Asp Leu Val Leu Met Asp Val Cys Met Pro
            820                 825                 830
Val Leu Asp Gly Leu Lys Ala Thr Arg Leu Ile Arg Ser Tyr Glu Glu
        835                 840                 845
Thr Gly Asn Trp Asn Ala Ala Ile Glu Ala Gly Val Asp Ile Ser Thr
    850                 855                 860
Ser Glu Asn Glu Gln Val Cys Met Arg Pro Thr Asn Arg Leu Pro Ile
865                 870                 875                 880
Ile Ala Met Thr Ala Asn Thr Leu Ala Glu Ser Ser Glu Glu Cys Tyr
                885                 890                 895
Ala Asn Gly Met Asp Ser Phe Ile Ser Lys Pro Val Thr Leu Gln Lys
            900                 905                 910
Leu Arg Glu Cys Leu Gln Gln Tyr Leu His
        915                 920
<210>      7
<211>      32
<212>      DNA
<213>      Artificial sequence
<220>
<221>
<222>
<233>      Primer No. 163
<400>      7
cgcggatcca ccatggtctg tgaaatggag ac                                32
<210>      8
<211>      32
<212>      DNA
<213>      Artificial sequence
<220>
<221>
<222>
<233>      Primer No. 154
<400>      8

ccgctcgagt cagtgcaaat actgttgcaa ac 32
<210> 9
<211> 31
<212> DNA
<213> Artificial sequence
<220>
<221>
<222>
<233> Primer No. 177
<400> 9
ggggtacctc agtgcaaata ctgttgcaaa c 31
<210> 10
<211> 33
<212> DNA
<213> Artificial sequence
<220>
<221>
<222>
<233> Primer No. 271
<400> 10
tacccggggg taccgtcgac ctgcaggcat gcc 33
<211> 11
<211> 32
<212> DNA
<213> Artificial sequence
<220>
<221>
<222>
<233> Primer No. 272
<400> 11
aaacgacggc cagtgaattc gagttcggca cc 32
<210> 12
<211> 31
<212> DNA
<213> Artificial sequence
<220>

<221>

<222>

<233>     Primer No. 162

<400>     12

cgcggatcca ccatgcttga ggcgagtact g                                    31